(19) Europäisches Patentamt
European Patent Office
Office européen des brevets

(11) EP 3 741 374 A1

(12) **EUROPEAN PATENT APPLICATION**
published in accordance with Art. 153(4) EPC

(43) Date of publication:
**25.11.2020 Bulletin 2020/48**

(21) Application number: **19741926.0**

(22) Date of filing: **18.01.2019**

(51) Int Cl.:
*A61K 31/7048* (2006.01) *A61K 45/06* (2006.01)
*A61P 35/00* (2006.01) *A61P 29/00* (2006.01)
*A61P 25/28* (2006.01) *A61P 3/10* (2006.01)
*A61P 9/00* (2006.01) *A61P 11/00* (2006.01)

(86) International application number:
**PCT/CN2019/072411**

(87) International publication number:
**WO 2019/141254 (25.07.2019 Gazette 2019/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.01.2018 CN 201810052779**

(71) Applicant: **Shenyang Fuyang Pharmaceutical Technology Co., Ltd.**
**Shenyang, Liaoning 110164 (CN)**

(72) Inventors:
- **XIA, Mingyu**
  **Shenyang, Liaoning 110164 (CN)**
- **ZHAO, Xiaofeng**
  **Shenyang, Liaoning 110164 (CN)**
- **JIANG, Xunlei**
  **Shenyang, Liaoning 110164 (CN)**
- **JIANG, Xundong**
  **Shenyang, Liaoning 110164 (CN)**

(74) Representative: **Hanna Moore + Curley**
**Garryard House**
**25/26 Earlsfort Terrace**
**Dublin 2, D02 PX51 (IE)**

# (54) MTOR INHIBITOR, PHARMACEUTICAL COMPOSITION AND USE THEREOF

(57) Disclosed are an mTOR inhibitor, a pharmaceutical composition and use thereof. The mTOR inhibitor includes one of carrimycin, isovalerylspiramycin I, isovalerylspiramycin II and isovalerylspiramycin III, or a combination of two or three of isovalerylspiramycin I, isovalerylspiramycin II and isovalerylspiramycin III. The pharmaceutical composition also includes a drug for treating and/or preventing diseases related to the mTOR pathway as a second active ingredient. The mTOR inhibitor has obvious inhibiting effect on cells of diseases related to mTOR pathway, and is used for preparing drugs for treating and/or preventing diseases related to the mTOR pathway.

EP 3 741 374 A1

## Description

## TECHNICAL FIELD

The present disclosure relates to the medicine field, and particularly relates to an mTOR inhibitor, a pharmaceutical composition and use thereof.

## BACKGROUND

Tumor is a kind of common and frequently-occurring disease. It is a new organism or neoplasm formed by abnormal clonal proliferation and differentiation caused by lose of normal regulation of growth and differentiation of organism tissue cells due to gene mutation under the long-term effect of various internal and external tumorigenic factors. Tumors are divided into benign tumors and malignant tumors, and malignant tumors are subdivided into three types: cancer derived from epithelial tissue, sarcoma derived from mesenchymal tissue and carcinosarcoma. The so-called term "cancer" is generally used to refer to all malignant tumors.

Malignant tumors are one of the major malignant diseases that threaten human health. They are currently the leading cause of death for the global population. According to the latest statistics, about 7.9 million people died of various types of cancers worldwide in 2007, accounting for 13% of the total deaths. More than 12 million cancer cases have been diagnosed, of which more than 72% of cancer patients and fatal cases have occurred in underdeveloped countries, and there is an increasing trend. In 2015, the number of global cancer deaths increased to 9 million, and it is expected to exceed 12 million people in 2030. At present, China has about 2.8 million cancer cases and more than 400,000 deaths each year, ranking the first among the causes of death from various diseases in China, and there is also a rising trend for this figure. With the acceleration of social life rhythm, increased competition pressure, and changes in human life style and environment, the incidence of cancer and deaths are increasing year by year. It has become a common disease and high incidence of modern society, which not only seriously affects the quality of life of patients, but also has brought a heavy economic and mental burden to families and society. It is also a major social problem that plagues the world. The treatment and prevention of cancer has always been one of the most urgent problems in the world.

Mammalian target of rapamycin (mTOR) is an atypical serine/threonine protein kinase and one of the members of the family of protein kinases related to phosphoinositide 3 kinase (PI3K). mTOR exists in the form of two complexes of mTORC1 and mTORC2 in vivo. mTOR can integrate various extracellular signals such as nutrition, energy and growth factors, participate in biological processes such as gene transcription, protein translation, ribosome synthesis and cytoskeletal synthesis, and play an extremely important role in cell growth, proliferation, apoptosis and metabolism. The initial stimulating factors for activation of the signaling pathway are mainly amino acids, various growth factors and hypoxia. mTOR exists in the form of two complexes of mTORC1 and mTORC2 in vivo.

The PI3K/AKT/mTOR pathway is a signaling pathway that regulates cell activity in mammals, and this pathway is important for cell survival, growth, and proliferation. Important mitogens (insulins, hormones, growth factors, etc.) activate molecules located on the side of the cell membrane close to the cytoplasm, thereby activating PI3K, an important molecule in the mTOR pathway. Activated PI3K promotes the conversion of phosphatidylinositol (4,5)-biphosphate (PIP2) to phosphatidylinositol(3,4,5)-triphosphate (PIP3), which binds to the PH domain of Akt and is also accompanied by phosphorylation of Akt by other kinases, which ultimately causes tuberous sclerosis complexes 1 and 2 (TSC1/2) to depolymerize or/and phosphorylate PRAS40 (proline rich AKT substrate 40000) to upregulate mTORC1. Abnormalities in the PI3K/AKT/mTOR signaling pathway frequently occur in a variety of types of tumors, including non-small cell lung cancer, endometrial cancer, cervical cancer, etc.

In addition, other environmental stressors can also regulate the mTOR signaling pathway. For example, long-term lack of indispensable oxygen in cell metabolism leads to energy deficiency, helps serine/threonine kinase liver kinase B1 (LKB1, liver kinase B1) or AMPK mediate mTORC1 inhibition. The availability of energy is also an important regulator of mTOR activity. AMP-activated protein kinase (AMPK) can be used as the "energy sensor" of mTORC1. When there lacks energy, the level of AMP in the cell rises, binds to AMPK, and activates AMPK through upstream kinases. Once activated, AMPK can phosphorylate TSC2, increase the decomposition process of production capacity and reduce energy-consuming synthesis processes, such as protein synthesis.

The most important feature of mTORC1 activation is protein synthesis. mTORC1 directly phosphorylates the hydrophobic group Thr389 of p70 ribosomal protein S6 kinase 1 (S6K1), thereby phosphorylating the subsequent protein by acting on PDK1. Phosphorylation of S6K1 activates several downstream substrates to promote the start of mRNA translation, including eIF4B, a positive regulator of the eIF4F complex. mTORC1 directly phosphorylates the downstream eukaryotic initiation factor 4E-binding protein 1 (4E-BP1). 4E-BP1 phosphorylation prevents it from binding to the cap-binding protein eIF4E, thus allowing it to participate in the activation of formation of eIF4F complex required for cap protein-dependent translation. Phosphorylation of S6K1 and 4E-BP1 activates the translation of mRNA, thereby increasing the level of activity of various effectors. mTORC1 has very important significance in the translation of mRNA. The

inhibitor of mTOR active site can completely inhibit the function of mTORC1, thereby reducing the protein synthesis in the cell. In addition to regulating protein synthesis, mTORC1 also controls the synthesis of lipids required for cell membranes. At the same time, mTORC1 also actively mediates cell metabolism and ATP synthesis.

mTORC2 consists of mTOR, mLST8, Deptor, Rictor (rapamycin-insensitive companion of mTOR), mammalian stress activated protein kinase interacting protein 1 (mSIN1) and Protor 1/2 (protein observed with Rictor-1/2). Through mSIN1 and protein kinase C-$\alpha$ (PKC$\alpha$), mTORC2 can participate in the regulation of actin and the formation of cytoskeleton. High expression of mTORC2 can promote cell survival, while low expression of mTORC2 induces apoptosis.

The key step of the mTORC2 pathway is to regulate and inhibit the conversion of PIP2 to PIP3 through protein phosphatase (PTEN, phosphatase and tensin homologue deleted on chromosome 10). mTORC1 can indirectly activate mTORC2 by activating ribosome biosynthesis and inhibiting autophagy-mediated ribosome turnover. mTORC2 activation will cause Akt 473 serine (Ser473) phosphorylation, Akt activation further induces phosphorylation of SIN1-T86, mTORC2 activity is enhanced, which promotes the formation of mTORC2 positive loop, but the inhibition of TSC1/2 complex will weaken the process.

In many human tumors, mTOR is abnormally activated. The use of mTOR inhibitors can effectively inhibit the abnormally activated PI3K/Akt/mTOR signaling pathway of various tumor cells such as lung cancer, breast cancer, pancreatic cancer, gastric cancer, melanoma, glioma, liver cancer, etc., thereby inhibiting the migration and invasion of tumor cells and epithelial mesenchymal transformation. A high percentage of cancer patients have mutations in the oncogenic pathway upstream of mTORC1, including the PI3K/AKT/mTOR pathway and the Ras/Raf/MEK/ERK pathway. Mutations in the above two pathways cause excessive activation of mTORC1. In addition, the common tumor suppressors TP53 and LBK1 are negative regulators of TSC1 and TSC2 upstream of mTORC1. The downstream factors of mTORC1 are also involved in tumorigenesis. The overexpression of eIF4E and S6K1 genes and proteins is present in many cancers, among which the phosphorylation of 4E-BP1 is the most critical. Some AKT and ERK-driven tumor cell lines are dependent on the phosphorylation of 4EBP. In addition, mTOR inhibitors can change the expression ratio of 4EBP and eIF4E, and thus have a strong inhibitory effect on proliferation of these cells.

mTORC2 signaling is also involved in cancer, largely due to its role in activating Akt. The activated Akt promotes proliferation processes such as glucose uptake and glycolysis, while also inhibiting apoptosis. In fact, some PI3K/Akt-induced tumors also depend on mTORC2 activity. PTEN is missing in mouse models of prostate cancer, as is PTEN in human prostate cancer cell lines.

Activation of PI3K/AKT/mTOR signal transduction pathway can inhibit apoptosis induced by various stimuli, promote cell cycle progression, cell survival and proliferation, and participate in angiogenesis, tumor invasion and metastasis, and play an important role in tumor formation. AKT can regulate multiple apoptosis-related proteins to inhibit apoptosis. Overexpression of AKT increases the expression of apoptosis protein inhibitory factor 1 (Dap-1) to play a role in inhibiting apoptosis. AKT can also transmit survival signals by phosphorylating mTOR and its downstream molecules S6K1 and 4E-BP1, inhibit P53-independent apoptosis, and promote cell survival. In recent years, it has been found in a variety of tumors that eIF-4E has cell transformation and anti-apoptotic activity in vitro, and over-expression of eIF-4E can protect cells from certain pre-apoptotic effects.

Extracellular regulated protein kinases (ERK), including ERK1 and ERK2, are the key to transmitting signals from surface receptors to the nucleus. Phosphorylation-activated ERK1/2 is translocated from the cytoplasm into the nucleus, which in turn mediates the transcriptional activation of Elk-1, ATF, Ap-1, c-fos and c-Jun, and participates in cell proliferation and differentiation, cell morphology maintenance, construction of cytoskeleton, apoptosis, and canceration of cells. ERKs regulate cell proliferation, differentiation, and survival, and they are downstream proteins of various growth factors (EGF, NGF, PDGF, etc.). ERK and its signaling pathways play a role of intermediation and signal amplification in tumor invasion and transfer process. On the one hand, it receives a large number of signals from growth factors, mitogens, environmental stimuli, etc. On the other hand, it acts on nuclear transcription factors such as AP-1 and NF-$\kappa$B, etc. through the ERK signal cascade reaction and regulates gene expression. The excessive activation of ERK can be found in many human cancers (such as oral cancer, melanoma, breast cancer, etc.). Its classic pathway is Ras/Raf/MER/ERK.

What is more worthy of attention is that there are multiple crosstalk phenomena between PI3K/AKT/mTOR and ERK/MAPKs signaling pathways to form complex interactions. The interaction between these two pathways may be mutual inhibition or mutual activation. PI3K/AKT/mTOR and ERK/MAPKs signaling pathways play an important role in the growth, proliferation, differentiation, invasion, metastasis and drug resistance of tumor cells. Their functions and regulatory mechanisms are extremely complex. Inhibiting one of these pathways cannot achieve the desired therapeutic effect, and the main reason is the complicated cross-talk relationship formed between them.

The mTOR inhibitors, such as rapamycin and its derivative "rapalogs", can specifically inhibit mTORC1, have a concentration and time-dependent inhibitory effect on the growth of various tumor cells, and can increase the sensitivity of tumor cells to chemotherapy drugs, induce the occurrence of apoptosis, and simultaneously produce a synergistic effect.

Carrimycin, also known as Bitespiramycin and Shengjimycin, is a new type of antibiotic with

4" isovalerylspiramycin as the main component formed by cloning 4"-isovaleryl transferase gene (4"-o-acyl-transferase) of the carbomycin-producing strain into the spiramycin-producing strain through transgenic technology, directionally acylating spiramycin 4"-OH, and adding isovaleryl side chain at 4" position under the collaboration between the Institute of Biotechnology of the Chinese Academy of Medical Sciences and the applicant.

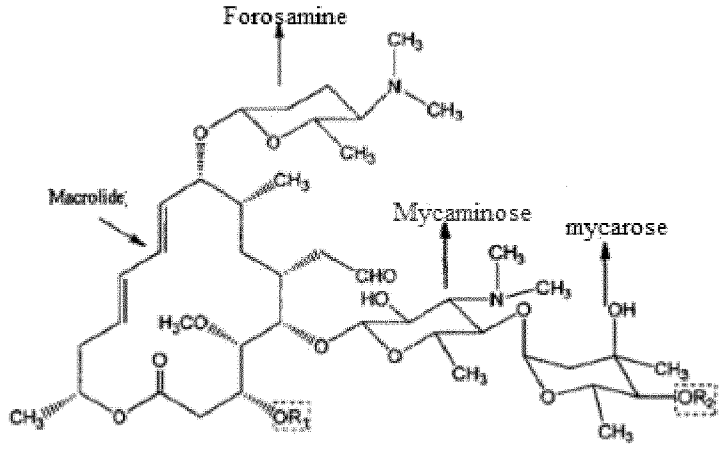

Formula (I)

Carrimycin is composed of a variety of spiramycin derivatives, with the total content of isovalerylspiramycins (I+II+III), the main active ingredient, not less than 60% and the total content of acylated spiramycin not less than 80%, and it is an acceptable pharmaceutical composition in pharmacy. The central structure is a 16-membered macrolide, which is connected with a molecule of forosamine, a molecule of mycaminose, and a molecule of mycarose. The main components of carrimycin, isovalerylspiramycins I, II, III, structurally differ from spiramycin in that the group attached to the 4" position of mycarose is isovaleryl instead of hydroxyl. The chemical structure of carrimycin is as shown in formula (1), and contains more than ten kinds of components. At present, the composition standard of the finished product of carrimycin is that isovalerylspiramycin III is ≥ 30%, the total ratio of isovalerylspiramycin I, II, III is ≥ 60%, the proportion of total acylated spiramycin is ≥ 80%, and the sum of other unknown components is ≤ 5%.

Carrimycin is a 16-membered macrolide antibiotic with active groups of carboxyl, alkoxy, epoxy, ketone and aldehyde groups and a pair of conjugated C=C, with a molecular weight of about 884-982. Due to the similar chemical structure, carrimycin and macrolide antibiotics have a lot in common: they are easily soluble in most organic solvents such as esters, acetone, chloroform, and alcohols, and are slightly soluble in petroleum ether, and insoluble in water; their molecular structures contain two dimethylamino groups and is weakly alkaline, and thus they are easily soluble in acidic aqueous solutions; they have a "negative solubility" quality that decreases in solubility with increasing temperature. Because the main component of carrimycin, isovalerylspiramycin, has a longer carbon chain at the 4" position, it has a poor hydrophilicity, and its solubility in water is less than that of spiramycin and 4"-acetylspiramycin.

Carrimycin is a white non-crystalline powder with a slight hygroscopicity, a specific rotation of about -80.8°, a maximum ultraviolet absorption wavelength of 231 to 232nm. It has a weak fluorescent chromophore itself, and will reacts as purple and produce strong purple fluorescence when encountered with concentrated sulfuric acid or hydrochloric acid, with the maximum absorbance at 231-232nm. This drug has good lipophilicity, strong tissue penetration ability, fast oral absorption, long-term maintenance in the body, and sustained post-antibiotic effect. According to the relationship between the efficacy and the chemical conformation, after the acylation of the macrolide antibiotic at the 4" position, its lipophilicity and in vivo activity are improved, the in vivo antibacterial activity and clinical treatment effect have been significantly improved, and the stability of the antibiotic in the body is also enhanced with the growth of the carbon chain of the 4" hydroxy ester, i.e., isovalerylspiramycin> butyrylspiramycin> propionylspiramycin> acetylspiramycin.

Preliminary in vivo and in vitro pharmacodynamic tests show that the drug not only has good antibacterial activity on most G+ bacteria, but also has certain effect on some G- bacteria, and various technical indexes are obviously superior to azithromycin, erythromycin, acetylspiramycin and midecamycin, especially has the strongest antibacterial activity on mycoplasma pneumoniae, and has certain antibacterial activity on erythromycin resistant bacteria, neisseria gonorrhoeae, pneumococcus, staphylococcus aureus, pseudomonas aeruginosa, bacillus influenzae, haemophilus influenzae, bacteroides fragilis, legionella pneumophilia, bacteroides thetaiotaomicron and clostridium perfringens, and has little cross resistance to erythromycin resistant staphylococcus aureus clinically. Carrimycin will be mainly used to

treat Gram-positive bacteria infectious diseases, especially upper respiratory infection, and may be used for urinary system infection, etc.

Pharmacokinetic research results show that the active components in carrimycin are mainly isovalerylspiramycins I, II and III. Carrimycin is rapidly metabolized into spiramycin after entering the body, and its oral absolute bioavailability is 91.6% on average based on the $AUC_{0-t}$ sum of the parent drugs isovalerylspiramycins I, II, III and the active metabolites spiramycins I, II and III. Literature reports that the absolute oral bioavailability of spiramycin is 30-40% in human body [Frydman AM et al J Antimicrob Chemother.1988, 22(suppl B):90-103]. This indicates that the structure of isovalerylspiramycin obviously improves the bioavailability of spiramycin, the active ingredient. The elimination of carrimycin is slower after a single dose, and $T_{1/2\beta}$ is between 23 and 27 hours.

The applicant has surprisingly found in his recent research that carrimycin, a single active ingredient of carrimycin or combination can inhibit PI3K/Akt/mTOR signaling pathway proteins, can be used as an mTOR inhibitor, has therapeutic effect on diseases related to PI3K/Akt/mTOR signaling pathway, and thus has important economic and social benefits.

The present disclosure has been made in view of this.

## SUMMARY

The technical problem to be solved by the present disclosure is to overcome the defects of the prior art and the present provide an mTOR inhibitor, a pharmaceutical composition and use thereof. The mTOR inhibitor provided by the present disclosure has obvious inhibiting effect on cells of diseases related to the mTOR pathway, provides theoretical basis for the application and clinical popularization of the mTOR inhibitor in preparing medicines for treating and/or preventing diseases related to the mTOR pathway, and has important economic benefits and social benefits.

In order to solve the above technical problems, the basic idea of the technical solution adopted by the present disclosure is as follows:

The first object of the present disclosure is to provide an mTOR inhibitor, the mTOR inhibitor comprises one of carrimycin, isovalerylspiramycin I, isovalerylspiramycin II and isovalerylspiramycin III, or a combination of two or three of isovalerylspiramycin I, isovalerylspiramycin II and isovalerylspiramycin III.

Carrimycin is a mixture of various active ingredients, including three active ingredients of isovalerylspiramycin I, isovalerylspiramycin II and isovalerylspiramycin III, as well as other impurities.

Each of carrimycin, isovalerylspiramycin I, isovalerylspiramycin II and isovalerylspiramycin III can be used as an mTOR inhibitor alone. Isovalerylspiramycin I, isovalerylspiramycin II and isovalerylspiramycin III can also be used in any combination.

Further, the mTOR inhibitor is an allosteric inhibitor or a catalytic inhibitor of proteins in a PI3K/Akt/mTOR signaling pathway.

Further, the catalytic inhibitor is a kinase inhibitor, e.g., an AKT inhibitor.

Further, the mTOR inhibitor is for inhibiting activation of mTORC1 and mTORC2.

Further, the mTOR inhibitor is for at least inhibiting activation of one or more of PI3K protein, AKT protein, mTOR protein, S6K1 protein and 4EBP1 protein in a PI3K/Akt/mTOR signaling pathway.

Further, the mTOR inhibitor is a drug selected from a group consisting an anti-tumor drug, a drug for treating diabetes, a drug for treating Alzheimer disease, and a drug for delaying senility, and the drug acts through an mTOR signaling pathway.

Further, the mTOR inhibitor is the anti-tumor drug acting through the mTOR signaling pathway, and at least for inhibiting activation of one or more of PI3K protein, AKT protein, mTOR protein, S6K1 protein and 4EBP1 protein in the PI3K/Akt/mTOR signaling pathway.

Further, the mTOR inhibitor is the drug for treating diabetes that acts through an mTOR signaling pathway, and at least for inhibiting activation of one or more of PI3K protein, AKT protein, mTOR protein, S6K1 protein and 4EBP1 protein in the PI3K/Akt/mTOR signaling pathway.

Further, the mTOR inhibitor is the drug for treating Alzheimer disease that acts through an mTOR signaling pathway, and at least for inhibiting activation of one or more of PI3K protein, AKT protein, mTOR protein, S6K1 protein and 4EBP1 protein in the PI3K/Akt/mTOR signaling pathway.

Further, the mTOR inhibitor is a drug for delaying senility that acts through an mTOR signaling pathway, and at least inhibits activation of one or more of PI3K protein, AKT protein, mTOR protein, S6K1 protein and 4EBP1 protein in the PI3K/Akt/mTOR signaling pathway.

The second object of the present disclosure is to provide a pharmaceutical composition, comprising the mTOR inhibitor as described in any of the above solution and a pharmaceutically acceptable carrier;

preferably, a dosage of the mTOR inhibitor is in a range from 1 to 10000 mg/kg; preferably from 10 to 5000 mg/kg, preferably from 50 to 1000 mg/kg, and more preferably from 100 to 500 mg/kg.

In another solution, the pharmaceutical composition comprises a first active ingredient and a second active ingredient, and the first active ingredient comprises the mTOR inhibitor as described above, and the second active

ingredient comprises a drug for treating and/or preventing diseases related to an mTOR pathway;
further, during preparation of a compound preparation, the dosage ratio of the first active ingredient and the second active ingredient is 1-99: 99-1, preferably 5-95: 95-5, more preferably 10-90: 90-10, and still more preferably 20-80: 80-20.

Further, the pharmaceutical composition comprises any pharmaceutically acceptable formulations; preferably, the formulations comprise powder, tablet, granule, capsule, solution, emulsion and suspension.

In the present disclosure, the active ingredient isovalerylspiramycin I in the mTOR inhibitor can be separated and prepared according to the method of the prior art, such as the method of Example 1 of CN101785778A.

The third object of the present disclosure is to provide a combination product, comprising a first medicament, and the first medicament comprises the mTOR inhibitor as described above or the pharmaceutical composition as described above.

Further, the combination product further comprises a second medicament.

Further, the second medicament comprises a drug for treating and/or preventing diseases related to the mTOR pathway.

The drugs for treating and/or preventing diseases related to an mTOR pathway in this solution refer to the main drugs for treating these diseases. For example, for diabetes, the second medicament can be insulin and its analogues, sulfonylurea secretagogues, metformins, $\alpha$-glucosidase inhibitors, thiazolidinedione derivative sensitizers, anisic acid derivative secretagogues, GLP-1 receptor stimulants, DPP-4 receptor stimulants and Chinese patent medicines.

In combined therapy, the dosage ratio of the first medicament and the second medicament is 1-99: 99-1, preferably 5-95: 95-5, more preferably 10-90: 90-10, and still more preferably 20-80: 80-20.

In combined therapy, the first and second medicaments are administered in any order. The first medicament can be used first, or the second medicament can be used first, or both medicaments can be used at the same time.

The fourth object of the present disclosure is to provide an anti-tumor drug, comprising an mTOR inhibitor comprising one of carrimycin, isovalerylspiramycin I, isovalerylspiramycin II and isovalerylspiramycin III, or a combination of two or three of isovalerylspiramycin I, isovalerylspiramycin II and isovalerylspiramycin III;
preferably, the mTOR inhibitor is an allosteric inhibitor or a catalytic inhibitor of proteins in a PI3K/Akt/mTOR signaling pathway;
preferably, the catalytic inhibitor is a kinase inhibitor;
preferably, the mTOR inhibitor at least inhibits activation of one or more of PI3K protein, AKT protein, mTOR protein, S6K1 protein and 4EBP1 protein in the PI3K/Akt/mTOR signaling pathway.

Many cell functions closely related to tumors, such as cell proliferation, cell cycle, protein synthesis, and cell migration, are controlled by the regulation of mTOR. It has been found that many tumors such as breast cancer, prostate cancer, and lung cancer have abnormal regulation of the mTOR signaling pathway. One of carrimycin, isovalerylspiramycin I, isovalerylspiramycin II, isovalerylspiramycin III, or a combination of two or three of isovalerylspiramycin I, isovalerylspiramycin II, isovalerylspiramycin III is used as an mTOR inhibitor for inhibiting the activity of mTORC1, which in turn affects its downstream target molecules regulated by mTORC1, including p70S6K, ATG13, 4EBP1, HIF-1, PGC-1$\alpha$, PPARr etc. When the activity of mTORC1 decreases, p70S6K is negatively regulated, cell growth is blocked, and ATG13 is no longer inhibited, thereby promoting cell apoptosis and autophagy. In addition to the cell cycle arrest, the inhibitor of the present disclosure can also cause tumor cell death through apoptosis and autophagy.

The fifth object of the present disclosure is to provide a drug for treating diabetes, comprising an mTOR inhibitor comprising one of carrimycin, isovalerylspiramycin I, isovalerylspiramycin II and isovalerylspiramycin III, or a combination of two or three of isovalerylspiramycin I, isovalerylspiramycin II and isovalerylspiramycin III;
preferably, the mTOR inhibitor is an allosteric inhibitor or a catalytic inhibitor of proteins in a PI3K/Akt/mTOR signaling pathway;
preferably, the catalytic inhibitor is a kinase inhibitor;
preferably, the mTOR inhibitor at least inhibits activation of one or more of PI3K protein, AKT protein, mTOR protein, S6K1 protein and 4EBP1 protein in the PI3K/Akt/mTOR signaling pathway.

In addition, mTOR can form different functional complexes (mTORC1 and mTORC2) to regulate insulin signaling pathway activity, affect islet $\beta$ cell development, apoptosis and insulin secretion, regulate secretion of hormones, such as ghrelin /nesfatin-1, closely related to glucose metabolism, and affect glucose uptake by peripheral tissues such as skeletal muscle and fat, etc. to regulate blood sugar in various ways. The mechanism of action of mTORC1 on insulin sensitivity is complex. On the one hand, growth factor can activate mTOR through classical PI3K-AKT signaling pathway, and on the other hand, the mTOR/S6K1 signal can reduce insulin sensitivity through negative feedback mechanism. The mTOR inhibitor of the present disclosure inhibits the hyperphosphorylation of mTOR and S6K1, reverses IRS serine phosphorylation in an insulin resistance state, enhances sugar absorption of adipocytes, and inhibits fat accumulation.

The sixth object of the present disclosure is to provide a drug for treating Alzheimer disease, comprising an mTOR inhibitor comprising one of carrimycin, isovalerylspiramycin I, isovalerylspiramycin II and isovalerylspiramycin III, or a combination of two or three of isovalerylspiramycin I, isovalerylspiramycin II and isovalerylspiramycin III;
preferably, the mTOR inhibitor is an allosteric inhibitor or a catalytic inhibitor of proteins in a PI3K/Akt/mTOR signaling

pathway;
preferably, the catalytic inhibitor is a kinase inhibitor;
preferably, the mTOR inhibitor is for at least inhibiting activation of one or more of PI3K protein, AKT protein, mTOR protein, S6K1 protein and 4EBP1 protein in a PI3K/Akt/mTOR signaling pathway.

In the nervous system, excessive activation of mTOR may lead to the occurrence of brain tumors. In addition, many evidences show that there are abnormalities in mTOR signaling pathway in some neurodegenerative diseases such as Alzheimer disease (AD), Parkinson's disease, Huntington's chorea, etc. These diseases all have a common feature: a large number of neurons are lost in certain areas of the brain, which may be related to abnormalities in mTOR pathway. AD is a neurodegenerative disease with progressive dementia as its main feature, which mainly occurs in the elderly. The most typical pathological features of AD are: accumulation of β-amyloid protein (Aβ) outside neurons forms senile plaques, highly phosphorylated proteins in neurons forms neurofibrillary tangles and loss of neurons and clinical manifestations are changes in learning and memory functions. Because the pathogenesis of AD is very complex, there are two different changes (up/down) in the mTOR pathway in the current research. Down-regulation of mTOR pathway can reduce the synthesis of some proteins with neurotoxic effects (e.g. tau protein), therefore mTOR inhibitors may become effective drugs for the treatment of neurodegenerative diseases.

The seventh object of the present disclosure is to provide a drug for delaying senility, comprising an mTOR inhibitor comprising one of carrimycin, isovalerylspiramycin I, isovalerylspiramycin II and isovalerylspiramycin III, or a combination of two or three of isovalerylspiramycin I, isovalerylspiramycin II and isovalerylspiramycin III;
preferably, the mTOR inhibitor is an allosteric inhibitor or a catalytic inhibitor of proteins in a PI3K/Akt/mTOR signaling pathway;
preferably, the catalytic inhibitor is a kinase inhibitor;
preferably, the mTOR inhibitor is for at least inhibiting activation of one or more of PI3K protein, AKT protein, mTOR protein, S6K1 protein and 4EBP1 protein in a PI3K/Akt/mTOR signaling pathway.

Inhibition of mTOR/S6K signaling pathway can delay senility, increase mitochondrial production and improve respiratory chain activity, reduce endoplasmic reticulum stress, and promote autophagy to remove damaged structures in cells. Inhibition of an mTOR inhibitor on mTOR/S6K signaling pathway can significantly enhance autophagy-related signaling pathways. After mTORC1 is inhibited, autophagy is enhanced, the ability to remove metabolic byproducts is enhanced, and the ultimate life is prolonged. In addition, mTOR is the maintainer of mitochondrial oxidative respiratory function, which promotes mitochondrial related gene expression, mitochondrial production and increases tissue oxygen consumption by up-regulating PPARr and PGC1 levels. The inhibition of mTORC1 can activate gene groups with protective function, reduce the damage caused by oxygen free radicals by limiting mitochondrial respiration, thus prolonging the life of the body.

The eighth object of the present disclosure is to provide use of the mTOR inhibitor or the pharmaceutical composition or the combination product as described above in preparation of a drug for treating and/or preventing diseases related to the mTOR pathway.

Further, the carrimycin, the isovalerylspiramycin I, the isovalerylspiramycin II or the isovalerylspiramycin III manipulates a metabolic microenvironment by targeting an mTOR, thereby inhibiting diseases related to the mTOR pathway.

Further, diseases related to the mTOR pathway are selected from at least one of age-related diseases, diseases related to transplant rejection, chronic inflammatory diseases, diseases related to glycogen storage, Huntington's chorea, malignant tumor, metastatic cancer, systemic lupus erythematosus, diseases related to inflammation and immune activation, diseases related to leukopenia, anemia, thrombocytopenia, diseases related to stent coating, renal insufficiency, obesity, diabetes, diseases related to nonalcoholic fatty liver, weight loss caused by diseases, polycystic kidney, Parkinson's disease and fibrosis.

Further, the age-related diseases are selected from a group consisting of sarcopenia, skin atrophy, muscle atrophy, brain atrophy, atherosclerosis, arteriosclerosis, emphysema, osteoporosis, osteoarthritis, hypertension, erectile dysfunction, dementia, Alzheimer disease, cataract, age-related macular degeneration, prostate cancer, stroke, life expectancy reduction, renal function impairment and age-related hearing loss, senility-related mobility disability, cognitive impairment, memory impairment, tendon stiffness, cardiac dysfunction such as myocardial hypertrophy and systolic and diastolic dysfunction, and immune function senility.

Further, the fibrosis comprises liver fibrosis, myocardial fibrosis, cardiovascular fibrosis, pulmonary fibrosis, pancreatic fibrosis, renal fibrosis or spleen fibrosis.

Further, the malignant tumor is selected from a group consisting of hematopoietic tumor of a lymphatic system, medullary hematopoietic tumor, mesenchymal cell-derived tumor, tumor of central and peripheral nervous systems, melanoma, seminoma, teratoma, osteosarcoma, xeroderma pigmentosum, keratoacanthoma, thyroid follicular cancer and Kaposi's sarcoma;
preferably, the hematopoietic tumor of a lymphatic system is selected from a group consisting of leukemia, acute lymphoid leukemia, acute lymphoblastic leukemia, B-cell lymphoma, T-cell lymphoma, Hodgkin's lymphoma, non-Hodgkin's lymphoma, hairy cell lymphoma, mantle cell lymphoma, myeloma and Birket's lymphoma; the medullary hematopoietic tumor comprises acute and chronic myelocytic leukemia, myelodysplastic syndrome and promyelocytic leukemia; the mesenchymal cell-derived tumor comprises fibrosarcoma and rhabdomyosarcoma; the tumor of central and peripheral nervous systems comprises astrocytoma, neuroblastoma, glioma and schwannoma.

Further, the malignant tumor further comprises bladder cancer, breast cancer, colon cancer, mesothelioma, kidney cancer, liver cancer, lung cancer, head and neck cancer, esophageal cancer, gallbladder cancer, ovarian cancer, pancreatic cancer, gastric cancer, lymphoma, cervical cancer, colon cancer, thyroid cancer, prostate cancer, skin cancer, and oral cancer.

Further, malignant tumor cells inhibited by the mTOR inhibitor comprise: human breast cancer cells MCF-7 and MDA-MB-231, human liver cancer cells HepG2, human non-small cell lung cancer cells A549, human large cell lung cancer cells H460 and H1299, human kidney clear cell adenocarcinoma cells 786-O, human renal cell adenocarcinoma cells 769-P, human glioma cells U251, human glioblastoma cells A172, human tissue lymphoma cells U937, human cervical cancer cells HeLa, human prostate cancer cells PC3, human pancreatic cancer cells PANC-1, human esophageal cancer cells TE-1, human gastric adenocarcinoma cells SGC-7901, human colon cancer cells HT-29, and human promyelocytic leukemia cells HL-60.

As the most preferable solution, the mTOR inhibitor is for inhibiting lung cancer caused by human non-small lung cancer cells A549.

According to the present disclosure, further in vivo tests show that the isovalerylspiramycin I has obvious inhibiting effects on the growth of mouse liver cancer cells $H_{22}$ and non-small cell lung cancer cells A549.

Treatment with mTOR inhibitors used in accordance with the present disclosure may be combined with one or more other cancer treatments including surgical therapy, radiotherapy (e.g., gamma-radiation, neutron beam radiotherapy, electron beam radiotherapy, proton therapy, brachytherapy and systemic radioisotope therapy, etc.), endocrine therapy, biological response modulator therapy (e.g., some of the names are interferon, interleukin, tumor necrosis factor (TNF), hyperthermia, cryotherapy, relief of adverse reactions of drugs (such as anti-emetic drugs) and other cancer chemotherapy drugs. The other drugs may be administered before, during or after the use of the mTOR inhibitor provided by the present disclosure, and may be administered in the same or different formulations, routes of administration, and dosage arrangements as the mTOR inhibitor provided herein.

The mTOR inhibitor and pharmaceutical composition of the present disclosure can be used together with other drugs to relieve side effects (e.g., inhibin, analgesic, antiemetic, G-CSF, GM-CSF, etc.), and/or with other suitable chemotherapeutic drugs. The other drugs include but are not limited to one or more of the following: anticancer alkylating or embedded drugs (such as nitrogen mustard, chlorambucil, cyclophosphamide, melphalan and ifosfamide); metabolic antagonist drugs (such as methotrexate); purine antagonist or pyrimidine antagonist (such as 6-mercaptopurine, 5-fluorouracil, cytarabine, capecitabine and gemcitabine); spindle toxins (such as vinblastine, vincristine, vinorelbine and paclitaxel); podophyllotoxin (such as etoposide, irinotecan, topotecan); antibiotics (such as doxorubicin, bleomycin and mitomycin); nitrosourea (such as carmustine and lomustine); inorganic ions (such as cisplatin, carboplatin, oxaliplatin or oxiplatin); enzymes (such as asparaginase); hormones (such as tamoxifen, leuprorelin acetate, flutamide and megestrol); proteasome inhibitors (such as Velcade, other proteasome inhibitors or other NF-kB inhibitors, including, for example, IkK inhibitors; other kinase inhibitors (such as Src, BRC/Abl, kdr, flt3, aurora-2 and glycogen synthase kinase 3 ("GSK-3"), EGF-R kinase (such as Iressa, Tarceva, etc.), VEGF-R kinase, PDGF-R kinase, etc.; antibodies, soluble receptors or other receptors that antagonize receptors or hormones involved in cancer (including EGFR, ErbB2, VEGFR, PDGFR and IGF-R, and drugs such as herceptin (or other anti -Her2 antibodies), avastin, erbitux, etc.).

Examples of other therapeutic drugs include allopurinol, alemtuzumab, hexamethylmelamine, amifostine, nastrozole, antibodies to prostate specific membrane antigen (e.g., MLN-591, MLN591RL, and MLN2704), arsenic trioxide, bexarotene, bleomycin, busulfan, capecitabine, Gliadel Wafer, celecoxib, chlorambucil, cisplatin-epinephrine gel, cladribine, cytarabine liposome, daunorubicin liposome, daunorubicin, daunomycin, dexrazoxane, docetaxel, doxorubicin, Elliott B solution, epirubicin, estramustine, etoposide phosphate, etoposide, exemestane, fludarabine, 5-fluorouracil, fulvestrant, gemcitabine, gemtuzumab-ozogamicin, goserelin acetate, hydroxyurea, idarubicin, edarubicin, demethoxydaunor ubicin, ifosfamide, imatinib mesylate, irinotecan (or other topoisomerase inhibitors, including antibodies such as MLN576(XR11576)), letrozole, folinic acid, levamisole folinic acid, daunorubicin liposomes, melphalan, L-PAM, mesna, methotrexate, methoxsalen, mitomycin C, mitoxantrone, MLN518 or MLN608 (or flt-3 receptor tyrosine kinase, PDFG-R, c-kit other inhibitors), itoxantrone, paclitaxel, pegademase, pentostatin, porfimer sodium, rituximab, talc, tamoxifen, temozolomide, teniposide, VM-26, topotecan, toremifene, 2C4 (or other antibodies interfering with HER2 mediated signaling), tretinoin, retinoic acid, valrubicin, vinorelbine or pamidronate or zoledronate or bisphosphonate compounds.

The mTOR inhibitor therapy in the present disclosure may be used together with one or more combinations of cytotoxic agents as part of a therapeutic regimen. The combination of cytotoxic agents is selected from a group consisting of: CHOPP (cyclophosphamide, doxorubicin, vincristine, prednisone and procarbazine); CHOP (cyclophosphamide, doxorubicin, vincristine and prednisone); COP (cyclophosphamide, vincristine, prednisone); CAP-BOP (cyclophosphamide, doxorubicin, procarbazine, bleomycin, vincristine and prednisone); m-BACOD (methotrexate, bleomycin, doxorubicin, cyclophosphamide, vincristine, dexamethasone and folinic acid); ProMACE-MOPP (prednisone, methotrexate, doxorubicin, cyclophosphamide, etoposide, folinic acid, mechlorethaminoxide, vincristine, prednisone and procarbazine); ProMACE-CytaBOM (prednisone, methotrexate, doxorubicin, cyclophosphamide, etoposide, folinic acid, cytarabine, bleomycin and vincristine); MACOP-B (methotrexate, doxorubicin, cyclophosphamide, vincristine, prednisone, bleomycin and folinic acid); MOPP (mechlorethaminoxide, vincristine, prednisone and procarbazine); ABVD (doxorubicin/doxorubicin, bleomycin, vinblastine and dacarbazine); MOPP (mechlorethaminoxide, vincristine, prednisone and procarbazine) and ABV (doxorubicin/doxorubicin, bleomycin and vinblastine) used alternately; MOPP (mechlorethaminoxide, vincristine, prednisone and procarbazine) and ABVD (doxorubicin/doxorubicin, bleomycin, vinblastine and dacarbazine) used alternately; ChlVPP (chlorambucil, vinblastine, procarbazine and prednisone); IMVP-16 (ifosfamide, methotrexate and etoposide); MIME (mitoguazone, ifosfamide, methotrexate and etoposide); DHAP (dexamethasone, High Dose cytaribine and cisplatin); ESHAP (etoposide, methylprednisolone, high-dose cytarabine and cisplatin); CEPP(B) (cyclophosphamide, etoposide, procarbazine, prednisone and bleomycin); CAMP (lomustine, mitoxantrone, cytarabine and prednisone); CVP-1 (cyclophosphamide, vincristine and prednisone); ESHOP (etoposide, methylprednisolone, high-dose cytarabine, vincristine and cisplatin); EPOCH (etoposide, vincristine and doxorubicin, used for 96 hours accompanied by large doses of cyclophosphamide and oral prednisone); ICE (ifosfamide, cyclophosphamide and etoposide), CEPP(B) (cyclophosphamide, etoposide, procarbazine, prednisone and bleomycin), CHOP-B (cyclophosphamide, doxorubicin, vincristine, prednisone and bleomycin), CEPP-B (cyclophosphamide, etoposide, procarbazine and bleomycin), and P/DOCE (epirubicin or doxorubicin, vincristine, cyclophosphamide and prednisone).

With the technical solution described above, the present disclosure has the following beneficial effects over the prior art:

1. The mTOR inhibitor of the present disclosure comprises one of carrimycin, isovalerylspiramycin I, isovalerylspiramycin II and isovalerylspiramycin III, or a combination of two or three of isovalerylspiramycin I, isovalerylspiramycin II and isovalerylspiramycin III. Each of the above active ingredients can inhibit the activity of certain proteins in a PI3K/Akt/mTOR signaling pathway respectively or in combination, so the mTOR inhibitor of the present disclosure has obvious inhibiting effect on cells of diseases related to an mTOR pathway, and provides a theoretical basis for its application and clinical promotion in preparing drugs for treating and/or preventing diseases related to the mTOR pathway.
2. The mTOR inhibitor of the present disclosure has especially good anti-tumor effect, has especially good curative effect on tumors such as breast cancer, liver cancer, lung cancer, lymphoma, cervical cancer, prostate cancer, colon cancer or leukemia, etc. and can inhibit tumor cell proliferation by inhibiting the protein activity in the PI3K/Akt/mTOR signaling pathway. It provides a theoretical basis for the application of the mTOR inhibitor in the preparation of antitumor drugs and its clinical promotion, and thus has important economic and social benefits.

In the following, specific embodiments of the present disclosure will be described in further detail with reference to the accompanying drawings.

## BRIEF DESCRIPTION OF THE DRAWINGS

The accompanying drawings are a part of the present disclosure to provide a further understanding of the present disclosure. The illustrative embodiments of the present disclosure and the description thereof are used to explain the present disclosure, but do not constitute an improper limitation of the present disclosure. Obviously, the drawings in the following description are only some embodiments. For those skilled in the art, other drawings can be obtained according to these drawings without creative work. In the drawings:

Fig. 1 shows quantitative results of levels of proteins related with a PI3K/Akt/mTOR signaling pathway such as p-PI3k/PI3K, p-AKT/AKT, p-mTOR/mTOR, p-S6K1/S6K1, p-4EBP1/4EBP1, and β-actin and phosphorylated proteins as determined by the Western blot method, among which, A1-A2 is the result of p-PI3k/PI3K, B1-B2 is the result of p-AKT/AKT, C1-C2 is the result of p-mTOR/mTOR, D1-D2 is the result of p-S6K1/S6K1, and E1-E2 is the result of p-4EBP1/4EBP1;
Fig. 2 shows expression levels of proteins related with a PI3K/Akt/mTOR signaling pathway such as p-PI3k/PI3K, p-AKT/AKT, p-mTOR/mTOR, p-S6K1/S6K1, p-4EBP1/4EBP1, β-actin, etc. as determined by the Western blot method;
Fig. 3 shows a growth inhibition result of A549 cells after 24h and 48h treatment with carrimycin;
Fig. 4 shows the expression levels of proteins related with the PI3K/AKT/mTOR signaling pathway of A549 cells after 24h and 48h treatment with carrimycin as determined by the Western blot method;
Fig. 5 shows a quantitative result of the effect of carrimycin on the expression level of proteins related with the

PI3K/AKT/mTOR signaling pathway of A549 cells as determined by the Western blot method;

Fig. 6 shows a result of flow cytometry for detecting the autophagy of A549 cells induced by carrimycin, wherein the positive rate of MDC staining increased after induction; among them, Fig. 6a is the result of A549 cells induced by carrimycin for 24h; Fig. 6b is the result of A549 cells induced by carrimycin for 48h;Fig. 7 shows quantitative detection results of P62 and LC3 expression levels after 24h and 48h treating A549 cells with carrimycin as determined by the Western blot method;

Fig. 8 shows a growth inhibition result of carrimycin on A549 cells after adding an autophagy inhibitor 3-MA;

Fig. 9 shows results of cell morphology changes after 24 and 48 hours treatment with carrimycin as observed by a phase contrast microscope;

Fig. 10 shows a flow cytometric measurement result of A549 cells treated by carrimycin for 24h and 48h after AV-PI staining;

Fig. 11 shows results of the levels of pro-caspse3, cpase3 and PARP proteins of A549 cells treated by carrimycin for 24h and 48h as detected by the Western blot method;

Fig. 12 shows a detection result of caspe3 enzyme activity in A549 cells;

Fig. 13 shows quantitative analysis results of HIF-1$\alpha$VEGF-A protein level after treating A549 cells with different concentrations of carrimycin for 24 hours and 48 hours;

Fig. 14 shows quantitative analysis results of Ras, Raf, p-ERK/ERK protein levels after treating A549 cells with different concentrations of carrimycin for 24 hours and 48 hours.

It should be noted that the drawings and the literal description are not intended to limit the scope of the inventive concept in any way, but to explain the inventive concept to those skilled in the art by referring to specific embodiments.

## DETAILED DESCRIPTION

In order to make the objects, the technical solutions and the advantages of the examples of the present disclosure clearer, the technical solutions of the examples will be described clearly and completely below by referring to the examples of the present disclosure. The following examples are intended to explain the present disclosure, but are not intended to limit the scope of the present disclosure.

### Example 1: tablet of isovalerylspiramycin I, isovalerylspiramycin II or isovalerylspiramycin III

Specification: 200mg/350mg
Prescription of the tablet core:

| | |
|---|---|
| isovalerylspiramycin I, isovalerylspiramycin II or isovalerylspiramycin III | 200g |
| microcrystalline cellulose | 110g |
| sodium starch glycolate | 22g |
| povidone $K_{30}$ (5%) | 15g |
| magnesium stearate | 3g |
| formulated into 1000 tablets | |

Prescription of the coating solution:

| | |
|---|---|
| Opadry II | 21g |
| Distilled water | proper amount |
| formulated into 105ml | |

The preparation process:

Preparation of the tablet core: the main drug and adjuvants respectively were made to pass through a 100-mesh sieve , and a prescription dosage of isovalerylspiramycin I, isovalerylspiramycin II or isovalerylspiramycin III, a prescription dosage of microcrystalline cellulose and a 1/2 prescription dosage of sodium starch glycolate were uniformly mixed, and then an aqueous solution of 5% povidone $K_{30}$ was added to prepare a soft material. An 18-mesh screen was used for granulating, and then the wet granules were dried at 60°Cunder a ventilated condition for 2h. After the wet granules were dried, a 18-mesh screen was used for dispersing the granules, then a 1/2 prescription dosage of sodium

starch glycolate and a prescription dosage of magnesium stearate were added. After the materials were uniformly mixed, and the mixture was tabletted with a shallow concave die of the diameter of 11mm, to obtain a drug containing tablet core with the tablet weight of 350mg and the hardness of 6.5kg.

Preparation of the coating solution: the required amount of Opadry II (white color) was weighed, the required amount of water was added into the preparation container in batches, the stirring speed was reduced after all of the water has been added till the spiral disappears, and the stirring was continued to be performed for 30min to obtain the coating solution.

Preparation of the film coated tablets: the tablet core was placed into a coating pan, the coating conditions were determined and coating was carried out with the host speed of 20r/min, the air intake temperature of 40°C, the air outtake temperature of 30°C, the atomization pressure of 0.02Mpa and the guniting flow rate of 1ml/min. And after a constant state was achieved, the coating was continuously to be sprayed for 1.5h until the surfaces of the tablets were smooth and uniform in color. The tablets were qualified which were in compliance with the inspecting standards of thin-film coating. The coating added the weight by approximately 5%.

**Example 2: tablet of isovalerylspiramycin I, isovalerylspiramycin II or isovalerylspiramycin III (calculated for 10000 tablets)**

Prescription:

| | |
|---|---|
| raw powder isovalerylspiramycin I, II or of isovalerylspiramycin III | 1000g |
| low-substituted hydroxypropyl cellulose | (5%) 92.5g |
| sodium starch glycolate (3%) | 55.5g |
| magnesium stearate (1%) | 18.5g |
| starch | the total weight subtracts the weights of the other raw materials and excipients |
| total weight | 1850g |

Preparation process: a proper amount of starch was weighed, diluted to a concentration of 15%, and heated to pasteto obtain an adhesive; the main material isovalerylspiramycin I, isovalerylspiramycin II or isovalerylspiramycin III and the excipients starch, low-substituted hydroxypropyl cellulose, sodium starch glycolate and magnesium stearate passed through 100-meshes sieve, respectively, and the required main material and the excipients were weighed according to the prescription amount. After the isovalerylspiramycin I, starch and low-substituted hydroxypropyl cellulose were fully and uniformly mixed, a starch paste with the starch concentration of 15% was used to prepare the mixture into a soft material which was granulated by a 14-mesh sieve, and granules were dried at 50-60°C to control the moisture content to be 3-5%. A 14-mesh sieve was used for dispersing the granules,, and then sodium starch glycolate and magnesium stearate were added to be mixed and the granules content was measured. The tablet weight was calculated according to the granules content, and the mixture was tabletted by using a Φ9mm shallow concave punch, and the difference in the weight of tablets was detected. And, after the product passed the test, the tablets were packaged.

**Example 3: capsule of isovalerylspiramycin I, isovalerylspiramycin II or isovalerylspiramycin III (calculated for 10000 granules)**

Prescription:

| | |
|---|---|
| raw powder of isovalerylspiramycin I, isovalerylspiramycin II isovalerylspiramycin III | or 1000g |
| starch | 1080 subtracts the weight of the raw powder of isovalerylspiramycin I |
| medicinal No. 3 capsule | 1000 granules |
| liquid paraffin | 50ml |

Preparation process: the main material isovalerylspiramycin I, isovalerylspiramycin II or isovalerylspiramycin III and the excipient medicinal starch were separately weighed according to the dosages of the process prescription,

and then fully mixed in a mixer for 1.5-2 hours. The data obtained by sampling and content testing should be substantially consistent with the theoretical data (the weight contained by each of the capsules was approximately 0.105g); and according to the operation requirements of a fully automatic encapsulating machine, the medicinal No. 3 capsule checked to be qualified and the raw materials well mixed were filled in a filling device; and the filled capsules were subjected to a difference test (within ±10%, and <0.3g), to obtain a capsule that has been checked to have a qualified dissolution rate. The capsules that meet the requirements after being tested were put into a polishing machine to be polished for 15-20 minutes with the liquid paraffin added, and then were were taken out to be tested by finished product packaging boxes.

**Example 4: dried syrup of isovalerylspiramycin I, isovalerylspiramycin II or isovalerylspiramycin III (calculated for 10000 bags)**

Prescription:

| | |
|---|---|
| raw powder of isovalerylspiramycin I isovalerylspiramycin II or isovalerylspiramycin III | 1250g |
| citric acid (0.5%) | 15g |
| sucrose | the total weight subtracts the weights of the other raw materials and excipients |
| total weight, approximately | 5000g |
| pigment (Curcumin) | approximately 1g |

Preparation process: the raw powder of isovalerylspiramycin I, isovalerylspiramycin II or isovalerylspiramycin III, citric acid and sucrose were respectively grinded into granules by using a jet-stream pulverizer , and 85% of the granules passed through 300-meshes sieve, 15% of the granules passed through 180-meshes sieve. Then the pulverized fine powder was weighed according to the prescription amount and fully mixed for 1-1.5 hours. The content was measured, the filling amount was calculated (the theoretical filling amount is 500mg per bag). Then the mixture was put into a bagging machine, aluminum foils paper was installed, and filling was carried out according to the operation requirements of a filling machine. The difference was allowed to be within ±5 %, and after the filling, the outer packaging was carried out after passing the inspection.

Example 5: granule preparation of isovalerylspiramycin I, isovalerylspiramycin **II** or isovalerylspiramycin III (calculated for 10000 bags)

Prescription:

| | |
|---|---|
| Raw powder of isovalerylspiramycin III | isovalerylspiramycin I, isovalerylspiramycin II or 1250g |
| sugar powder | 20000g |
| dextrin | 9000g |
| 5% PVP-$K_{30}$ | proper amount |

Preparation process: the raw powder of isovalerylspiramycin I, isovalerylspiramycin II or isovalerylspiramycin III, sugar powder and dextrin passed through 120-meshes sieve, and the isovalerylspiramycin I, sugar powder and dextrin were weighed according to the prescription amount and uniformly mixed. And the above materials uniformly mixed were made into a soft material with a 5% PVP-$K_{30}$ mucilage. Then the soft material was granulated with a swinging granulation machine, dried at 70°C and subjected to granule dispersion, and the resulting granules were subpackaged after being qualified for inspection.

**Example 6: freeze-dried powder injection of isovalerylspiramycin I, isovalerylspiramycin II or isovalerylspiramycin III**

500mg of raw powder of isovalerylspiramycin I, isovalerylspiramycin II or isovalerylspiramycin III was uniformly mixed with an equimolar amount of hexanedioic acid, and the mixture was dissolved in 5ml water, to obtain a faint-yellow clear solution with a pH between 4.6 and 5.6. Then 40mg of mannitol was added as a lyophilization proppant into the

faint yellow clear solution, and after being frozen rapidly at a low temperature for 9h, the material was freeze-dried to obtain a faint yellow loose mass, which was dissolved in 10 ml of sterile water before being used.

**Example 7: freeze-dried powder injection of isovalerylspiramycin I and isovalerylspiramycin II**

250mg of raw powder of isovalerylspiramycin I and 250mg of raw powder of isovalerylspiramycin II were mixed uniformly with an equimolar amount of hexanedioic acid, and the mixture was dissolved in 5ml water to obtain a faint-yellow clear solution with a pH between 4.6 and 5.6. Then 40mg of mannitol was added as a lyophilization proppant into the faint yellow clear solution, and after being frozen rapidly at a low temperature for 9h, the material was freeze-dried to obtain a faint yellow loose mass, which was dissolved in 10 ml of sterile water before being used.

**Example 8: freeze-dried powder injection of carrimycin**

500mg of raw powder of carrimycin was mixed uniformly with an equimolaramount of hexanedioic acid, and the mixture was dissolved in 5ml water to obtain a faint-yellow clear solution with a pH of 4.6-5.6. Then 40mg of mannitol was added as the lyophilization proppant into the faint yellow clear solution, and after being frozen rapidly at a low temperature for 9h, the material was freeze-dried to obtain a faint yellow loose mass, which was dissolved in 10 ml of sterile water before being used.

**Test Example 1: Bioassay of Antitumor Activity**

The object of the assay is to evaluate the in vitro cell proliferation inhibition or cytotoxic activity of the tested sample.

Cell strains:

Human breast cancer cells MCF-7 and MDA-MB-231, human hepatoma cell HepG2, human non-small cell lung cancer cell A549, human cell lung cancer cells H460 and H1299, human renal clear cell adenocarcinoma cell 786-O, human renal cell adenocarcinoma cell 769-P, human glioma cell U251, human glioblastoma cell A172, human tissue lymphoma cell U937, human cervical cancer cell HeLa, human prostate cancer cell PC3, human pancreatic cancer cell PANC-1, human esophageal cancer cell TE-1, human gastric gland cancer cell SGC7901, human colon cancer cell HT-29 and human promyelocytic leukemia cell HL-60, commercially available from American Type Culture Collection (ATCC, Manassas, VA, USA).

Reagents:

RPMI1640 nutrient solution, MEM nutrient solution, DMEM low-sugar nutrient solution, fetal bovine serum commercially available from the Gibco company in the United States, and trypsin, glutamine, penicillin, streptomycin, dimethyl sulfoxide (DMSO), methyl thiazolyl tetrazolium (MTT) commercially available from the Sigma company in the United States.

Instruments:

Carbon-dioxide incubator (Sanyo, Japan), enzyme linked immunosorbent assayer (Tecan, Austria), 96-well culture plate (Corning, USA), and inverted microscope (Motic, China).
The operation steps are as follows:

Adherent cells:

MCF-7, MDA-MB-231, HepG2, A549, H460, H1299, 786-O, 769-P, U251, A172, HeLa, PC3, PANC-1, TE-1, SGC7901, HT-29 were adherent tumor cells. The adherent tumor cell in the logarithmic growth phase were selected and digested with trypsin, then were prepared into a cell suspension of 4-5 $\times 10^4$/ml by using a culture medium containing 10% of fetal bovine serum. And the cell suspension was inoculated into the 96-well culture plate with 100$\mu$l per well, culturing at 37°C with 5% $CO_2$ for 24h. The experimental group was replaced with a new culture solution containing different concentrations of the tested sample carrimycin, while the control group was replaced with a culture solution containing the same volume of solvent. Each group was set up with 3 parallel wells and cultured at 37°C for 48 h with 5% $CO_2$. After the supernatant was removed, the wells were washed carefully by using PBS for 3 times. And 100$\mu$L of a newly formulated culture medium containing 0.5 mg/ml of MTT was added to each well for continuous incubation at

37°C for 4h. After the supernatant was removed carefully, , 150mL of DMSO was added to each well, and after the material was mixed uniformly by using a microoscillator for 10 min, and the value of the optical density was measured by using a microplate reader at 492nm.

Suspension cells:

U937 and HL-60 were suspension cells, and cells in the logarithmic growth phase were selected and prepared into a cell suspension of $2\times10^5$/ml by using a culture medium RPMI 1640 containing 10% of fetal bovine serum. And the cell suspension was inoculated into the 96-well culture plate with 50μl per well, and the 96-well culture plate was cultured at 37°C with 5% $CO_2$ for 24h. 50μL culture solution containing different concentrations of the tested sample carrimycin was added in the experimental group, while a culture solution containing the same volume of solvent was added in the control group. Each group was set up with 3 parallel wells that were cultured at 37°C for 48 h with 5% $CO_2$. And 10μL of a newly formulated culture medium containing 5 mg/ml of MTT was added into each well for continuous incubation at 37°C for 4h. The crystals were dissolved in 100 μL of a triple solution (SDS 10 g, 10 MHCl 0.1 mL, isobutanol 5 mL, diluted to 100 ml with distilled water) and incubated at 37°C for 12 h; and the value of the optical density was measured by using a microplate reader at 492nm.

Assessment of the result: the inhibition rate of drugs on tumor cell growth was calculated according to the following formula:

$$\text{Tumor cell growth inhibition rate (\%)} = [A_{492}\ \text{(negative control)} - A_{492}\ \text{(dosing group)}] / A_{492}\ \text{(negative control)} * 100\%$$

The median inhibition concentration ($IC_{50}$) of the sample was determined from this formula.

Results: Human breast cancer cells MCF-7 and MDA-MB-231, human liver cancer cell HepG2, human non-small cell lung cancer cell A549, human large cell lung cancer cells H460 and H1299, human renal clear cell adenocarcinoma cell 786-O, human renal cell adenocarcinoma cell 769-P, human glioma cell U251, human glioblastoma cell A172, human tissue lymphoma cell U937, human cervical cancer cell HeLa, human prostate cancer cell PC3, human pancreatic cancer cell PANC-1, human esophageal cancer cell TE-1, human gastric gland cancer cell SGC-7901, human colon cancer cell HT-29, and human promyelocytic leukemia cell HL-60 were used as the experimental objects. The results of in vitro antiproliferative activity evaluation on samples are shown in Table 1 below:

Table 1. Inhibition of carrimycin on the proliferation of tumor cells

| Cell Strain | $IC_{50}$ (μg/mL) | Cell Strain | $IC_{50}$ (μg/mL) |
|---|---|---|---|
| MCF-7 | 11.2± 1.5 | A172 | 11.2±2.0 |
| MDA-MB-231 | 14.8 ± 1.0 | U937 | 12.4 ± 0.8 |
| HepG2 | 8.8 ± 2.7 | HeLa | 11.9 ± 2.8 |
| A549 | 15.4±2.1 | PC3 | 7.4 ± 2.4 |
| H460 | 7.7 ± 0.9 | PANC-1 | 9.1 ± 1.3 |
| H1299 | 12.7 ± 1.7 | TE-1 | 7.8 ± 2.1 |
| 786-O | 18.0 ± 2.5 | SGC-7901 | 8.2 ±1.6 |
| 769-P | 7.6 ±3. 7 | HT-29 | 12.1 ± 2.7 |
| U251 | 6.9 ± 1.2 | HL-60 | 17.5 ± 1.7 |

The existing results show that the samples all show good antiproliferative activity against the tested cells.

**Test Example 2: Isovalerylspiramycin I Inhibits PI3k/AKT/mTOR Pathway**

Cell strains:

A549 cells were purchased from American Type Culture Collection (ATCC, Manassas, VA, USA). The cells were cultured in a DMEM medium containing 10% fetal bovine serum, 2% glutamine and penicillin (100U/ml) in an

incubator at 37°C with 5% $CO_2$. The cells used in the experiment were all cells in logarithmic phase.

Reagents:

Rapamycin was purchased from Sigma Company (St. Louis, MO, USA)
The operation steps were as follows:
A549 was an adherent cell, cells in logarithmic growth phase was selected as the experimental group. And isovalerylspiramycin I with different concentrations (0.05, 5, 17, 50μg/ml) and control drug Rapamycin (20μg/ml) was added in the experimental group, and the the experimental group were cultured in an incubator at 37°C with 5% $CO_2$ for 24h, then cells were collected, and then the Western blot method was proceed.

Western blot:

1 preparation of working solution
1) 30% polyacrylamide solution:

| Name of the reagent | Dosage |
|---|---|
| acrylamide | 290 g |
| methylene bisacrylamide | 10 g |
| $ddH_2O$ | 1,000 ml |

$ddH_2O$ was added to a constant volume of 1,000 ml, placed in a brown bottle and stored at 4°C.
2) Tris buffer solution: after Tris base was completely dissolved in deionized water, the pH value of the solution was adjusted by HCl.

Separation gel buffer solution: 1.5 MTris-HCl (pH 8.8);
Stacking gel buffer solution: 1 MTris-HCl (pH 6.8).

3) Sodium dodecyl sulfate (SDS): a 10% storage solution prepared with deionized water was stored at room temperature for later use.
4) Ammonium persulfate (AP): a small amount of 10% (w/v) stock solution prepared with deionized water was stored at 4°C, which should be prepared fresh every other week because ammonium persulfate will decompose slowly.
5) 5×Tris-glycine electrophoretic buffer solution

| This solution | Name of the reagent | Dosage | was stored in refrigerator and was into a solution with for use. |
|---|---|---|---|
| a at 4°C, diluted 1 ×buffer $ddH_2O$ | Tris | 7.55 g | |
| | 10% (w/v) SDS | 25 mL | |
| | glycine | 47 g | |
| | $ddH_2O$ | Metered to 500 mL | |

6) Cell Lysis Solution: 50 mMHepes (pH 7.4), 1% Triton-X 100, 100 mMNaF, 1 mM EDTA, 1 mM EGTA, 2 mM sodium orthovanadate, 1 mM PMSF, 10 mg/ml aprotinin, 10 mg/ml leupeptin, 10 mg/ml pepstatin A.
7) 5 × Loading buffers:

| Name of the reagent | Dosage (mL) |
|---|---|
| Tris-HCL (pH 6.8) | 0.6 |
| glycerol (50%) | 5 |
| SDS (10%) | 2 |
| β -mercaptoethanol | 0.5 |
| bromophenol blue (1%) | 1 |

8) Transfer buffer:

| Name of the reagent | Dosage |
|---|---|
| Tris-Base | 4.55 g |
| glycine | 21.65 g |
| $ddH_2O$ | 1200 mL |
| methanol | 300 mL |

9) Blocking buffer:

| Name of the reagent | Dosage |
|---|---|
| skimmed milk powder | 5 g |
| PBST | 100 mL |

2. Protein electrophoresis

1) 60-100μl of lysate was added to each tube of cells, and was subjected to ice bath in a refrigerator at 4°C for 1h; the solution was centrifuged for 10-15 min at 12,000r/min, the supernatant was suck out to a 0.5ml EP tube; after the quantitative protein was measured with Bio-Rad, 5×loading buffers were added, and the solution was subjected to boiling water bath for 3-5min and then was cryopreserved at -80°C.
2) 15%, 12% or 10% acrylamide separation gel was prepared according to Tab. 2-1, and was then mixed with various ingredients successively; once TEMED and AP were added, the mixture was quickly rotated and poured between the two glass plates of the electrophoresis tank, leaving the space required for pouring the stacking gel.

The separation gel was covered with a layer of isopropyl alcohol, and the gel was placed vertically at room temperature for about 30 min.

3) After the separation gel was completely polymerized, the isopropyl alcohol was removed, and the gel was washed with deionized water for 10 times; water was absorbed with filter paper, a stacking gel was prepared according to Tab.2-2, and a clean sample comb was immediately inserted after adding the stacking gel into electrophoresis tank.
4) After the stacking gel was completely polymerized, the electrophoresis instrument was filled with 1×electrophoretic buffer solution, samples were added into sample holes according to a predetermined sequence to start electrophoresis, the voltage was 50-60V when the samples were located at the stacking gel, and the voltage was adjusted to 100-140V when the samples swam to the separation gel.

3. Western blot

1) After the SDS polyacrylamide gel electrophoresis was completed, the gel was placed in the transfer buffer and soaked for 30 min. 8 pieces of Whatman 3 MM filter paper and 1 piece of nitrocellulose membrane were cut out, which were consistent with the size of the gel. The nitrocellulose membrane was immersed in methanol for 1 min, and the nitrocellulose membrane was immersed in deionized water to remove air bubbles.
2) The gel was stacked into a "sandwich" shape and inserted into the transfer cell. The gel was connected to the cathode on one side, 100mA for 3 h.
3) The transferred nitrocellulose membrane entered the Ponceau S staining solution for 5-10min, and was shaken gently during the period. After the protein band appeared, it was rinsed with deionized water several times, and the position of the standard protein molecular weight was marked with waterproof ink.
4) The membrane was placed in a petri dish with a blocking solution and blocked for 2h.
5) The blocking solution containing a first antibody was added at 0.1 $ml/cm^2$ and stayed overnight at 4°C (dilution multiples of various antibodies are as shown in Tab.2-2)
6) The first antibody was recovered, the membrane was rinsed with PBST 3 times for 10 min each time, and was then transferred to a solution containing 150 mM NaCl and 50 mM Tris-HCl (pH 7.5) and shaken for 10 min.
7) A phosphate-free and sodium azide-free blocking solution containing a second antibody was added at 0.1 ml/cm2, wherein the second antibody labeled with horseradish peroxidase was diluted 1,000 times.

8) It was rinsed with 150 mM NaCl and 50 mmol/L Tris-HCl (pH 7.5) solution for 3 times for 10 min each time. Color development was carried out in a dark room with an ECL kit, and pictures were scanned and saved.

Table 2-A The solution for preparing electrophoretic separation gel

| Solution composition | 15 ml gel | 30 ml gel |
|---|---|---|
| 10% | | |
| water | 5.9 | 11.9 |
| 30% Acrylamide solution | 5.0 | 10.0 |
| 1.5 mMTris (pH 8.8) | 3.8 | 7.5 |
| 10% SDS | 0.15 | 0.3 |
| 10% Ammonium persulfate | 0.15 | 0.3 |
| TEMED | 0.006 | 0.012 |
| 12% | | |
| water | 4.9 | 9.9 |
| 30% Acrylamide solution | 6.0 | 12.0 |
| 1.5 mMTris (pH 8.8) | 3.8 | 7.5 |
| 10% SDS | 0.15 | 0.3 |
| 10% Ammonium persulfate | 0.15 | 0.3 |
| TEMED | 0.006 | 0.012 |
| 15 % | | |
| water | 3.4 | 6.9 |
| 30% Acrylamide solution | 7.5 | 15.0 |
| 1.5 mMTris (pH 8.8) | 3.8 | 7.5 |
| 10% SDS | 0.15 | 0.3 |
| 10% Ammonium persulfate | 0.15 | 0.3 |

Table 2-B The solution for preparing 5% stacking gel of Tris-glycine SDS polyacrylamide gel electrophoresis

| Solution composition | Different volumes (ml) required volume of each component in gel solution (ml) | | | | |
|---|---|---|---|---|---|
| | 4 | 5 | 6 | 8 | 10 |
| water | 2.7 | 3.4 | 4.1 | 5.5 | 6.8 |
| 30%Acrylamide solution | 0.67 | 0.83 | 1.0 | 1.3 | 1.7 |
| 1.0 mMTris (pH 6.8) | 0.5 | 0.63 | 0.75 | 1.0 | 1.25 |
| 10% SDS | 0.04 | 0.05 | 0.06 | 0.08 | 0.1 |
| 10%Ammonium persulfate | 0.04 | 0.05 | 0.06 | 0.08 | 0.1 |
| TEMED | 0.004 | 0.005 | 0.006 | 0.008 | 0.01 |

Table 2-C Antibodies for protein electrophoresis

| Antibodies | Type of IgG | Dilution |
|---|---|---|
| p-PI3K | rabbit polyclonal IgG | 1:1000 |
| PI3K | rabbit polyclonal IgG | 1:500 |
| p-AKT | rabbit polyclonal IgG | 1:1000 |
| AKT | rabbit polyclonal IgG | 1:500 |
| p-mTOR | rabbit polyclonal IgG | 1:1000 |
| mTOR | rabbit polyclonal IgG | 1:500 |
| p-S6K 1 | rabbit polyclonal IgG | 1:2000 |
| S6K1 | rabbit polyclonal IgG | 1:1000 |
| p-4EBP1 | rabbit polyclonal IgG | 1:1000 |

(continued)

| Antibodies | Type of IgG | Dilution |
|---|---|---|
| 4EBP1 | rabbit polyclonal IgG | 1:1000 |

Results:

After the isovalerylspiramycin I acted on A549 cells for 24 hours, the PI3K/Akt/mTOR signaling pathway protein and its phosphorylation type levels were investigated by the Western Blot method. The results were shown in Figures 1 and 2.

The effects of isovalerylspiramycin I on the expression of the protein levels of PI3K, AKT, mTOR and mTOR substrates S6K1, 4EBP1, and their activated types p-PI3K, p-AKT, p-mTOR, p-S6K1, p-4EBP1 were shown in Figs. 1 and 2. The results showed that isovalerylspiramycin I can inhibit the protein activation of the PI3K/AKT/mTOR signaling pathway, especially the activation of PI3K protein, 4EBP1 protein, mTOR protein, and inhibit expression of Akt protein and S6K1 protein and expression of their activated proteins.

In addition, as can be seen from Figs. 1 and 2, compared with rapamycin, the mTOR inhibitor isovalerylspiramycin I of the present disclosure has more excellent inhibitory effects on AKT, S6K1 and 4EBP1, indicating that the mTOR inhibitor isovalerylspiramycin I of the present disclosure can play an excellent PI3K/Akt/mTOR signaling pathway inhibitory effect, providing theoretical basis for the application and clinical promotion of the mTOR inhibitor in the preparation of drugs for treating and/or preventing diseases related to the mTOR pathway, and having important economic and social benefits.

In addition, the applicant also used carrimycin, isovalerylspiramycin II and isovalerylspiramycin III alone or two or three of isovalerylspiramycin I, isovalerylspiramycin II and isovalerylspiramycin III in combination to act on A549 cells for 24 hours, and then examined the levels of PI3K/Akt/mTOR signaling pathway protein and its phosphorylation types by the Western Blot method, and the results were similar to those in Test Example 2, i.e., it can inhibit the activation of PI3K protein, 4EBP1 protein and mTOR protein, inhibit the expression of AKT protein and S6K1 protein and the expression of their activated proteins, which will not be described in detail here.

**Test Example 3: Carrimycin Inhibits Proliferation of Non-small Cell Lung Cancer A549**

(1) MTT assay was used to detect the effect of carrimycin on the survival rate of tumor cells in vitro

A549 cells were inoculated into a 96-well plate at a density of $4\times10^3$ cells /mL, with 100 $\mu$L per well, and cultured for 24h or 48 h. Different concentrations of carrimycin were added in to the wells, the 96-well plate was placed in an incubator at 37°C with 5% $CO_2$ for continuous culture for different times. 100 $\mu$L of MTT with a concentration of 5 mg/mL MTT was added to each well for culture for 2-3 h, and the supernatant was sucked out and discarded. 150 $\mu$L DMSO was added, and shaken with a micro oscillator for 10 min to completely dissolve the crystals. The light absorption value (A value) of each well was detected with a microplate reader, wherein the emission wavelength was 492 nm. At the same time, rapamycin was used as a positive control, and the growth inhibition rate of the drug to cells was calculated according to the following formula: inhibition rate (%)=[$A_{492}$ (control)-$A_{492}$ (carrimycin)]/[$A_{492}$ (control)-$A_{492}$ (blank)] $\times$ 100%

The results showed that carrimycin could inhibit A549 cell proliferation in a concentration-dependent manner with $IC_{50}$ of 24.88 $\mu$g/mL and 17.84 $\mu$g/ml for 24h and 48 h, respectively.

(2) Carrimycin can inhibit PI3K/AKT/mTOR pathway

Abnormality occurs in a PI3K/AKT/mTOR signaling pathway frequently in various types of tumors. PI3K is an intracellular phosphatidylinositol kinase. PI3Ks protein family is involved in the regulation of cell proliferation, differentiation, apoptosis, glucose transport and other cell functions. AKT (protein kinase B) is a major effector downstream of PI3K, and can activate mTORC1 by phosphorylating mTOR directly or by inactivating TSC2 (tuberous sclerosis complex 2). mTORC1 directly phosphorylates downstream eukaryotic translation initiation factor binding protein 4E-BP1 and ribosomal protein S6 kinase S6K1, thereby regulating biosynthesis and proliferation of cells.

A549 cells were treated with different concentrations of carrimycin for 24h and 48h, and the mTOR protein level and its upstream and downstream protein levels in the cells were investigated by the Western Blot method. The results showed that after acting on A549 cells for 24h, carrimycin above 5 $\mu$g/ml can reduce the protein phosphorylation level of mTOR and downstream 4E-BP1 and S6K1, and also reduce the phosphorylation level of PI3K and AKT.

After 5, 17, 50 $\mu$g/ml of carrimycin treated A549 cells for 48h, the phosphorylation level of mTOR was significantly

reduced and the phosphorylation level of proteins downstream of mTOR was significantly inhibited. However, 5, 17 μg/ml of carrimycin increased the phosphorylation levels of PI3K and AKT, while 50 μg/ml of carrimycin could significantly reduce the phosphorylation levels of PI3K and AKT.

The above results proved that carrimycin inhibited cell protein synthesis by inhibiting the phosphorylation level of mTOR and downstream proteins, thus inhibiting A549 cell proliferation. The results showed that carrimycin of moderate and low concentrations inhibited mTORC1 to a higher degree, and there was negative feedback activation of mTORC2 after 48 h. However, carrimycin of a large concentration could be used as a dual inhibitor for both mTORC1 and mTORC2 without causing negative feedback activation (shown in Figs. 4 and 5).

(3) Carrimycin Induces Autophagy in A549 Cells

Method: Fluorescence microscopy and flow cytometry were used to detect autophagy

Dansylcadaverine (MDC) is a fluorescent pigment and an eosinophilic stain. It is usually used to detect the aggregation of specific marker stain acid lysosomes formed by autophagy, which can reflect the level of autophagy to some extent.

A549 cells in logarithmic growth phase were inoculated into a 6-well plate with $2\times10^5$ cells per well. After 24h of culture, the culture solution was discarded, different drugs were added to act for 24h or 48h, followed by washing with PBS once, a culture solution of equal volume containing 0.05 mM MDC was added, and then incubation was performed for 20 min at 37°C in the dark; washing was performed once with PBS, and observation was performed with a fluorescence microscope or detection was performed by flow cytometry.

Results: mTORC1 can inhibit autophagy by binding a ULK1 complex. Therefore, we investigated the autophagy level of cells treated with carrimycin for 24h and 48 h. MDC staining results showed that the cells in the control group emitted uniform green fluorescence. With the increase of concentration of carrimycin, obvious bright green fluorescent aggregated particles appeared in the cells. Flow cytometry results showed that MDC staining positive rate increased after drug treatment, and the results were consistent at 24 h and 48 h (Fig. 6).

LC3 is an autophagy marker protein. When autophagy occurs in cells, the level of transformation from LC3 type I to type II in cells will increase significantly. P62 is the substrate of autophagy. When autophagy occurs in cells, P62 mediates the binding of autophagy substrate and autophagy, and then it is encapsulated into lysosomes together with autophagy substrate and degraded. Therefore, when autophagy occurs in cells, the expression level of P62 in cells will decrease. Western blot analysis showed that the addition of carrimycin could reduce the level of autophagy substrate P62 and increase the transformation of autophagy related protein LC3 type I to type II, with the same results at 24 h and 48 h (Fig.7). The above results all prove that carrimycin can induce A549 cells to autophagy.

Since autophagy has dual effects on tumor cells, it can protect cells as well as kill cells. Therefore, we used 3-MA, an inhibitor of autophagy, to investigate the role of carrimycin-induced autophagy in A549 cell death. The addition of 3-MA autophagy inhibitor reduced the inhibitory effect of carrimycin on A549 cell growth (Fig. 8), indicating that carrimycin-induced autophagy inhibited A549 cell proliferation.

(4) Carrimycin Induces Apoptosis in A549 Cells

Method: Annexin V is a $Ca^{2+}$ dependent phospholipid binding protein with a molecular weight of 35 kDa, which can specifically bind Phosphatidylserine (PS). Phosphatidylserine is mainly distributed on the inner side of the cell membrane. When the cell apoptosis occurs, the cell will evert phosphatidylserine to the cell surface, i.e. the outer side of the cell membrane. A FITC labeled Annexin V probe, namely Annexin V-FITC, can specifically bind these everted phosphatidylserines, and the apoptosis of cells can be detected very simply and directly by a flow cytometry or a fluorescence microscope.

Propidium Iodide (PI) is a nucleic acid dye that cannot pass through normal cell membranes. When cells are in the middle or late stages of necrosis or apoptosis, the cell loses membrane integrity. Propidium Iodide enters the cell and binds with nucleic acid in the nucleus. A flow cytometry or a fluorescence microscopy can be used to detect and reflect the information of the complete state of the cell membrane. Therefore, Annexin V-FITC and PI were used together to distinguish cells in different apoptosis stages.

A549 cells in logarithmic growth phase were inoculated into a 6-well plate with $2\times10^5$ cells per well. After 24 h of culture, the culture solution was discarded, different drugs were added for 24 h or 48 h, followed by washing with PBS once; a prepared apoptosis staining solution (250μl Binding buffer, 7.5μl Annexin V-FITC, 10μl Propidium Iodide, which were mixed uniformly) was added, and the cells were incubated at room temperature in dark for 15 min, followed by fluorescence microscope observation and flow cytometry detection.

Results: In order to confirm the mechanism of carrimycin on A549 cells, AV-PI staining was used to investigate and flow cytometry was used to distinguish normal cells, early apoptotic cells, late apoptotic cells and necrotic cells.

Caspase family plays a very important role in the process of mediating cell apoptosis, wherein caspase3, as the downstream executive protein of apoptosis, is related to DNA fragmentation, chromatin condensation and apoptotic body formation. Therefore, we investigated the protease activity and protein level of caspase3 using kits and the Western blot method.

The results showed that A549 cells treated with carrimycin for 24h showed green fluorescence at 17 and 50 μg/ml, but no red fluorescence. Flow cytometry showed that cells with 50 μg/ml concentration showed early apoptosis (Fig. 10), caspase3 protein level did not increase significantly, and PARP prototype was not sheared (Fig. 11A). The A549 cells treated with carrimycin for 48h began to shrivel in cytoplasm and appeared apoptotic bodies with the increase of concentration (Fig. 9). AV-PI results showed that obvious green fluorescence appeared at 5 and 17 μg/ml, and obvious red fluorescence appeared at 50 μg/ml nucleus. Flow cytometry showed that the number of early apoptotic cells at 5 and 17 μg/ml increased and a large number of late apoptotic cells appeared at 50 μg/ml (Fig. 10). The conversion of caspase3 from a precursor to an activated form increased, PARP as a substrate was sheared (Fig. 11B), and the enzyme activity of caspase3 increased (Fig. 12), proving that apoptosis occurred in the cells.

(5) Carrimycin Reduces HIF -1α Protein Level

Hypoxia and other factors such as insulin and growth factor can induce the expression of HIF-1α, while overactivated mTOR can also activate the expression of HIF -1α from a transcription level under the condition of sufficient oxygen, thus leading to transcription of downstream vascular endothelial growth factor VEGF and other angiogenic genes, which has the effects of promoting vascular permeability increase, extracellular matrix degeneration, vascular endothelial cell migration, proliferation and angiogenesis.

Western blot analysis showed that the protein levels of vascular biosynthesis proteins HIF -1α and VEGF-A were significantly decreased after treating A549 cells with carrimycin for 24 and 48 h (Fig. 13), which suggests that carrimycin may inhibit tumor cell proliferation by inhibiting the protein level of A549 hypoxia inducible factor.

(6) Effect of carrimycin on ERK Signal Transduction Pathway

Ras/Raf/MEK/ERK signaling pathway is one of the most important signaling pathways that transmit extracellular signals to the nucleus and it plays a key role in regulating cell survival, colonization, differentiation, apoptosis, metabolism and other functions.

A549 cells treated with carrimycin for 24 h can increase Ras, Raf protein level and ERK protein phosphorylation level. At the same time, A549 cells treated with carrimycin for 48 h can increase Ras and Raf protein levels. Drugs of medium concentration can significantly increase ERK phosphorylation level, while drugs of high concentration can reduce ERK phosphorylation level. The results are shown in Fig. 14.

(7) Effect of carrimycin on Cell Cycle of A549 Cells

The cell cycle distribution of A549 treated with different concentrations of carrimycin for 24 h and 48 h was detected by PI flow cytometry. It was found that drug treatment for 24 h had no obvious effect on cell cycle. After 48 h of drug treatment, compared with the control group, the S-phase cells of 1.7 μg/ml, 5 μg/ml, 17 μg/ml and 50 μg/ml increased by 1.86%, 9.39%, 4.75% and 24.38%, respectively. To sum up, treatment with carrimycin for 48h induces S-phase arrest of A549 cells in a concentration-dependent manner.

As can be seen from the above test examples, after adding carrimycin to A549 cells for 24 h, the phosphorylation level of mTOR was significantly reduced, and the phosphorylation levels of eukaryotic translation initiation factor binding protein 4E-BP1 and ribosomal protein S6 kinase 1 S6K1 downstream of mTOR were significantly inhibited. By reducing the protein biosynthesis of tumor cells, the proliferation rate of tumor cells was slowed down, and the phosphorylation level of PI3K was lowered, and the phosphorylation level of AKT was not significantly affected. After treating A549 cells with carrimycin for 48 h, the protein phosphorylation level of mTOR pathway could still be down-regulated, but the PI3K/AKT pathway was activated in a feedback manner at a medium concentration, resulting in a significant increase in the phosphorylation level of PI3K and AKT. However, the phosphorylation levels of PI3K and AKT proteins were re-inhibited at a high concentration (50 μg/ml). Therefore, it is boldly speculated that carrimycin may be a dual inhibitor of mTORC1 and mTORC2. Under the condition of small and medium concentrations, carrimycin has strong inhibition on mTORC1, but there is a strong negative feedback regulation after 48 h of drug action; under the condition of a high concentration, it also has certain inhibitory effect on mTORC2.

By observing A549 cells treated with carrimycin for 48 h using a phase contrast microscope, cell shrinkage, chromatin condensation and formation of obvious apoptotic bodies and apoptotic florets were observed. JC-1 staining results showed that with the increase of drug concentration, the proportion of cells exhibiting green fluorescence gradually increased, indicating that carrimycin could reduce mitochondrial membrane potential of A549 cells in a concentration-dependent manner. We used the Western blot method to detect the level of apoptosis-related proteins. Procaspase-3 protein level decreased and cleaved-caspase3 protein level increased. Meanwhile, the caspase-3 substrate PARP was degraded, indicating that apoptosis of A549 cells induced by carrimycin activated caspase cascade reaction. The level of Bax increased and the levels of Bcl-XL and Bcl-2 decreased. Therefore, treatment with carrimycin for 48 h could inhibit the proliferation of tumor cells by inducing apoptosis of A549 cells. The above data showed that the apoptosis phenomenon was not obvious after 24 hours of treatment with carrimycin, while there was an obvious concentration-dependent apoptosis phenomenon after 48 hours, and early apoptosis changed to late apoptosis.

Carrimycin, as an inhibitor of mTOR, can significantly improve the transformation from LC type I to type II of A549 cells and down-regulate the protein level of autophagy substrate P62. MDC staining and flow cytometry also prove the increase of autophagy. After the addition of an autophagy inhibitor 3-MA, the inhibitory effect of carrimycin on A549 cells was reduced, which proves that the autophagy of A549 cells induced by carrimycin is a damaging autophagy, i.e., carrimycin inhibits proliferation of A549 cells by increasing the level of autophagy of the cells, while obvious autophagy phenomenon occurred at 24 h and 48 h. Before apoptosis occurs, carrimycin may mainly inhibit cell survival by inducing autophagy. In the present disclosure, the protein phosphorylation level of ERK was significantly increased after the A549 cell was treated with carrimycin for 24 hours, so it was considered that inhibition of PI3K/AKT/mTOR pathway activated Ras/Raf/MEK/ERK pathway.

In a word, carrimycin can inhibit proliferation and induce apoptosis of human non-small cell lung cancer A549 in a concentration-dependent manner, and can be used as a dual inhibitor for both of mTORC1 and mTORC2. At medium and low concentrations, mTORC1 is mainly inhibited, which will cause negative feedback activation of PI3K and AKT, while at high concentrations, mTORC2 can be inhibited, and the protein phosphorylation level of PI3K and AKT can be lowered. It can induce A549 cells to autophagy, and autophagy plays the role of killing cells. It can cause S-phase arrest of A549 cells. In addition, carrimycin also activates the Ras/Raf/ERK pathway, the Ras and Raf protein levels are up-regulated, and the phosphorylation level of ERK is increased.

The above description is only preferred embodiments of the present disclosure, and is not intended to limit the present disclosure in any way. Although the present disclosure has been disclosed in the preferred embodiments, it is not intended to limit the present disclosure. Any person skilled in the art can make some changes or modifications to the technical content of the above tips as equivalent embodiments without departing from the scope of the technical solution of the present disclosure. However, any simple modifications, equivalent changes and modifications made to the above embodiments according to the technical essence of the present disclosure are still within the scope of the technical solution of the present disclosure.

## Claims

1. An mTOR inhibitor, comprising one of carrimycin, isovalerylspiramycin I, isovalerylspiramycin II and isovalerylspiramycin III, or a combination of two or three of isovalerylspiramycin I, isovalerylspiramycin II and isovalerylspiramycin III.

2. The mTOR inhibitor according to claim 1, wherein, the mTOR inhibitor is an allosteric inhibitor or a catalytic inhibitor of proteins in a PI3K/Akt/mTOR signaling pathway;
preferably, the catalytic inhibitor is a kinase inhibitor;
preferably, the mTOR inhibitor is for inhibiting activation of mTORC1 and mTORC2;
preferably, the mTOR inhibitor is for at least inhibiting activation of one or more of PI3K protein, AKT protein, mTOR protein, S6K1 protein and 4EBP1 protein in the PI3K/Akt/mTOR signaling pathway.

3. The mTOR inhibitor according to claim 1 or 2, wherein, the mTOR inhibitor is a drug selected from a group consisting an anti-tumor drug, a drug for treating diabetes, a drug for treating Alzheimer disease, and a drug for delaying senility, and the drug acts through an mTOR signaling pathway.

4. The mTOR inhibitor according to claim 3, wherein, the mTOR inhibitor is the anti-tumor drug acting through the mTOR signaling pathway, and at least for inhibiting activation of one or more of PI3K protein, AKT protein, mTOR protein, S6K1 protein and 4EBP1 protein in a PI3K/Akt/mTOR signaling pathway.

5. The mTOR inhibitor according to claim 3, wherein, the mTOR inhibitor is the drug for treating diabetes that acts through an mTOR signaling pathway, and at least for inhibiting activation of one or more of PI3K protein, AKT protein, mTOR protein, S6K1 protein and 4EBP1 protein in a PI3K/Akt/mTOR signaling pathway.

6. The mTOR inhibitor according to claim 3, wherein, the mTOR inhibitor is the drug for treating Alzheimer disease

that acts through an mTOR signaling pathway, and at least for inhibiting activation of one or more of PI3K protein, AKT protein, mTOR protein, S6K1 protein and 4EBP1 protein in a PI3K/Akt/mTOR signaling pathway.

**7.** The mTOR inhibitor according to claim 3, wherein, the mTOR inhibitor is the drug for delaying senility that acts through an mTOR signaling pathway, and at least for inhibiting activation of one or more of PI3K protein, AKT protein, mTOR protein, S6K1 protein and 4EBP1 protein in a PI3K/Akt/mTOR signaling pathway.

**8.** A pharmaceutical composition, comprising the mTOR inhibitor according to claim 1 or 2 and a pharmaceutically acceptable carrier, and/or active ingredients;
preferably, a dosage of the mTOR inhibitor is in a range from 1 to 10000 mg/kg; preferably from 10 to 5000 mg/kg, preferably from 50 to 1000 mg/kg, and more preferably from 100 to 500 mg/kg;
preferably, the pharmaceutical composition comprises a first active ingredient and a second active ingredient, the first active ingredient comprises the mTOR inhibitor according to claim 1 or 2, and the second active ingredient comprises a drug for treating and/or preventing diseases related to an mTOR pathway;
preferably, the pharmaceutical composition comprises any pharmaceutically acceptable formulations, and preferably, the formulations comprise powder, tablet, granule, capsule, solution, emulsion and suspension.

**9.** A combination product, comprising a first medicament, wherein the first medicament comprises the mTOR inhibitor according to claim 1 or 2 or the pharmaceutical composition according to claim 8;
preferably, the combination product further comprises a second medicament;
preferably, the second medicament comprises a drug for treating and/or preventing diseases related to the mTOR pathway.

**10.** Use of the mTOR inhibitor according to claim 1 or 2 or the pharmaceutical composition according to claim 8 or the combination product according to claim 9 in preparation of a drug for treating and/or preventing diseases related to an mTOR pathway;
preferably, the carrimycin, the isovalerylspiramycin I, the isovalerylspiramycin II or the isovalerylspiramycin III, or a combination of two or three of the isovalerylspiramycin I, the isovalerylspiramycin II or the isovalerylspiramycin III is targeted at an mTOR to manipulates a metabolic microenvironment to inhibit diseases related to the mTOR pathway;
preferably, the diseases related to the mTOR pathway are at least one selected from a group consisting age-related diseases, diseases related to transplant rejection, chronic inflammatory diseases, diseases related to glycogen storage, Huntington's chorea, malignant tumor, metastatic cancer, systemic lupus erythematosus, diseases related to inflammation and immune activation, diseases related to leukopenia, anemia, thrombocytopenia, diseases related to stent coating, renal insufficiency, obesity, diabetes, diseases related to nonalcoholic fatty liver, weight loss caused by diseases, polycystic kidney, Parkinson's disease and fibrosis;
preferably, the age-related diseases are selected from a group consisting of sarcopenia, skin atrophy, muscle atrophy, brain atrophy, atherosclerosis, arteriosclerosis, emphysema, osteoporosis, osteoarthritis, hypertension, erectile dysfunction, dementia, Alzheimer disease, cataract, age-related macular degeneration, prostate cancer, stroke, life expectancy reduction, renal function impairment and age-related hearing loss, senility-related mobility disability, cognitive impairment, memory impairment, tendon stiffness, cardiac dysfunction comprising myocardial hypertrophy and systolic and diastolic dysfunction, and immune function senility;
preferably, the fibrosis comprises liver fibrosis, myocardial fibrosis, cardiovascular fibrosis, pulmonary fibrosis, pancreatic fibrosis, renal fibrosis or spleen fibrosis;
preferably, the malignant tumor is selected from a group consisting of hematopoietic tumor of a lymphatic system, medullary hematopoietic tumor, mesenchymal cell-derived tumor, tumor of central and peripheral nervous systems, melanoma, seminoma, teratoma, osteosarcoma, xeroderma pigmentosum, keratoacanthoma, thyroid follicular cancer and Kaposi's sarcoma;
preferably, the hematopoietic tumor of a lymphatic system is selected from a group consisting of leukemia, acute lymphoid leukemia, acute lymphoblastic leukemia, B-cell lymphoma, T-cell lymphoma, Hodgkin's lymphoma, non-Hodgkin's lymphoma, hairy cell lymphoma, mantle cell lymphoma, myeloma and Birket's lymphoma; the medullary hematopoietic tumor comprising acute and chronic myelocytic leukemia, myelodysplastic syndrome and promyelocytic leukemia; the mesenchymal cell-derived tumor comprising fibrosarcoma and rhabdomyosarcoma; the tumor of central and peripheral nervous systems comprising astrocytoma, neuroblastoma, glioma and schwannoma;
preferably, the malignant tumor further comprises bladder cancer, breast cancer, colon cancer, mesothelioma, kidney cancer, liver cancer, lung cancer, head and neck cancer, esophageal cancer, gallbladder cancer, ovarian cancer, pancreatic cancer, gastric cancer, lymphoma, cervical cancer, thyroid cancer, prostate cancer, skin cancer, and oral cancer;

preferably, malignant tumor cells inhibited by the mTOR inhibitor comprise: human breast cancer cells MCF-7 and MDA-MB-231, human liver cancer cells HepG2, human non-small cell lung cancer cells A549, human large cell lung cancer cells H460 and H1299, human kidney clear cell adenocarcinoma cells 786-O, human renal cell adenocarcinoma cells 769-P, human glioma cells U251, human glioblastoma cells A172, human tissue lymphoma cells U937, human cervical cancer cells HeLa, human prostate cancer cells PC3, human pancreatic cancer cells PANC-1, human esophageal cancer cells TE-1, human gastric adenocarcinoma cells SGC-7901, human colon cancer cells HT-29, and human promyelocytic leukemia cells HL-60;
preferably, the mTOR inhibitor is for inhibiting human non-small cell lung cancer cells A549.

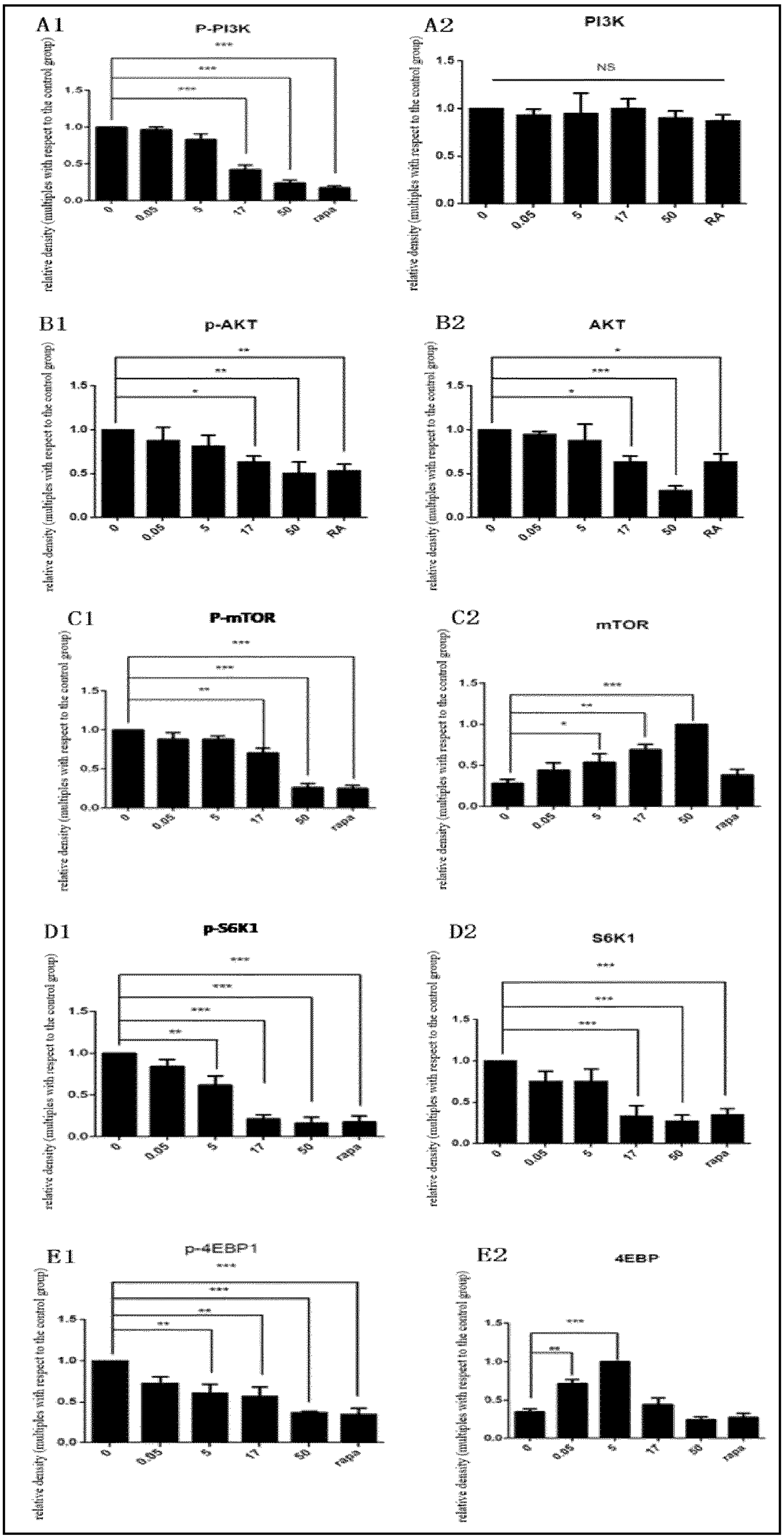
A1
P-PI3K
A2
PI3K
NS
B1
p-AKT
B2
AKT
C1
P-mTOR
C2
mTOR
D1
p-S6K1
D2
S6K1
E1
p-4EBP1
E2
4EBP
relative density (multiples with respect to the control group)
0
0.05
5
17
50
rapa
RA

Fig. 1

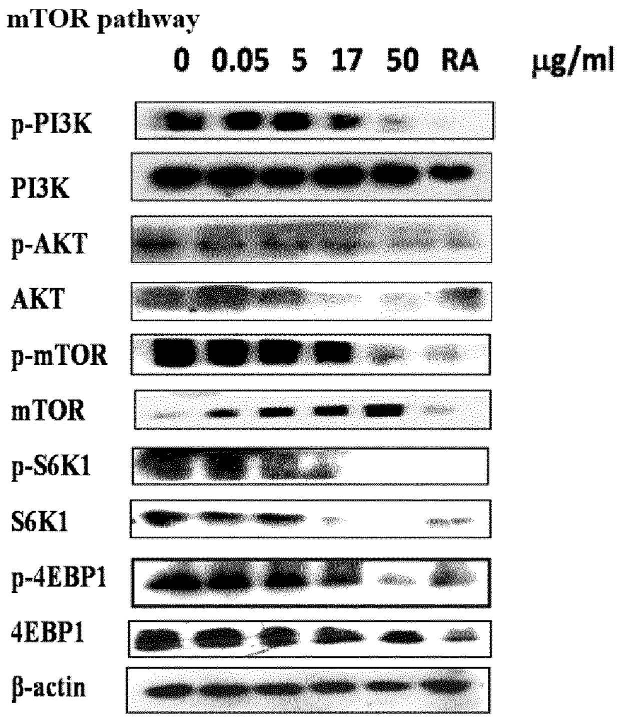


Fig. 2

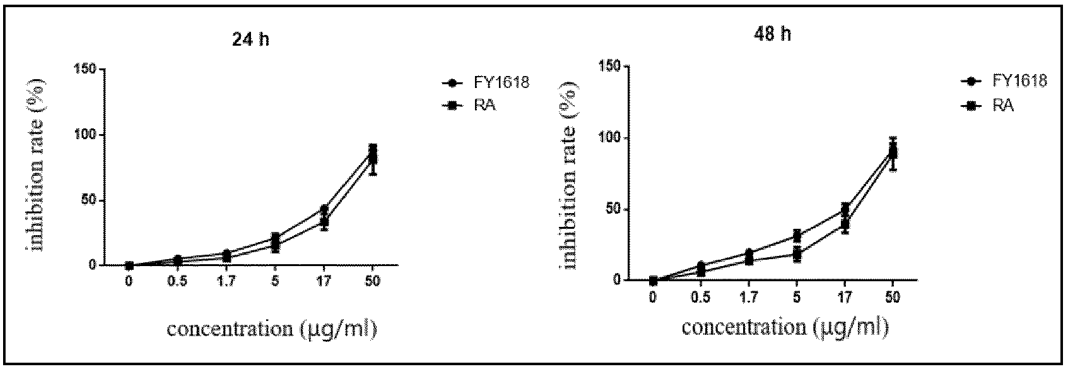


Fig. 3

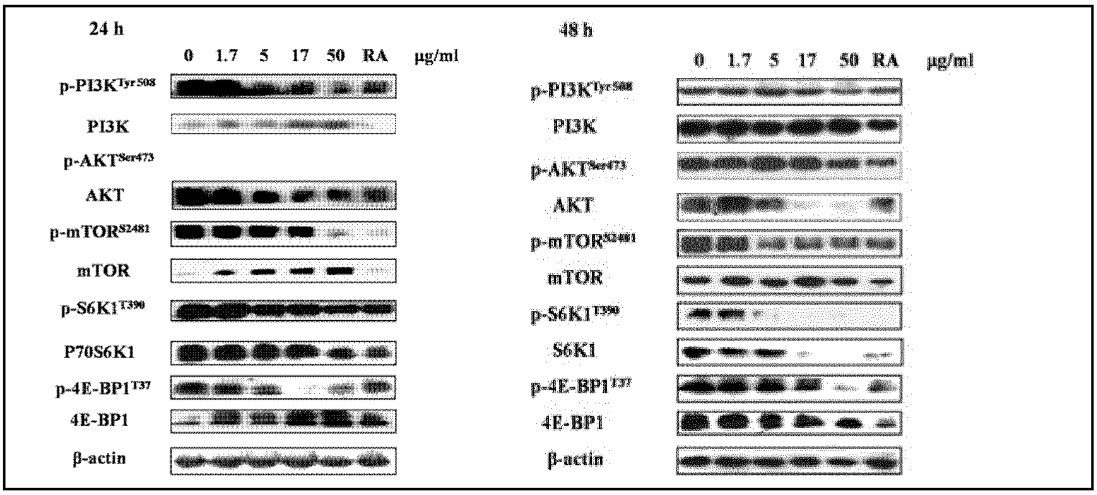


Fig. 4

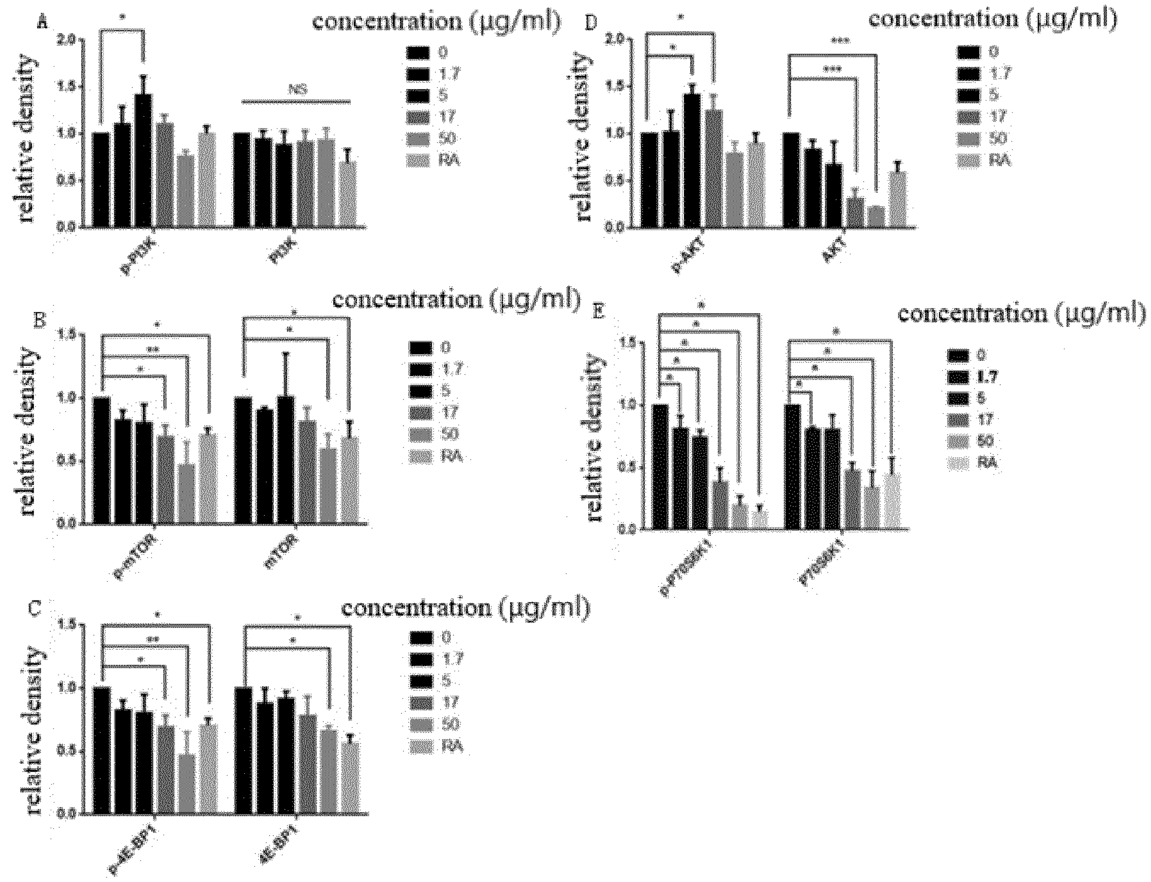
A
B
C
D
E
concentration (μg/ml)
relative density
0
1.7
5
17
50
RA
NS
p-PI3K
PI3K
p-AKT
AKT
p-mTOR
mTOR
p-P70S6K1
P70S6K1
p-4E-BP1
4E-BP1

Fig. 5

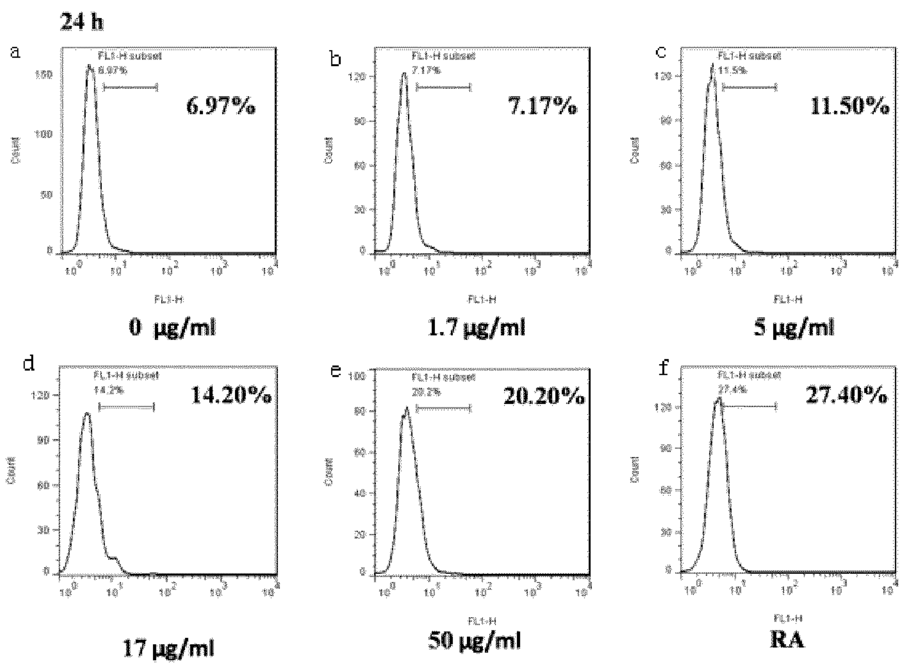
24 h
a
b
c
d
e
f
6.97%
7.17%
11.50%
14.20%
20.20%
27.40%
0 μg/ml
1.7 μg/ml
5 μg/ml
17 μg/ml
50 μg/ml
RA

Fig. 6a

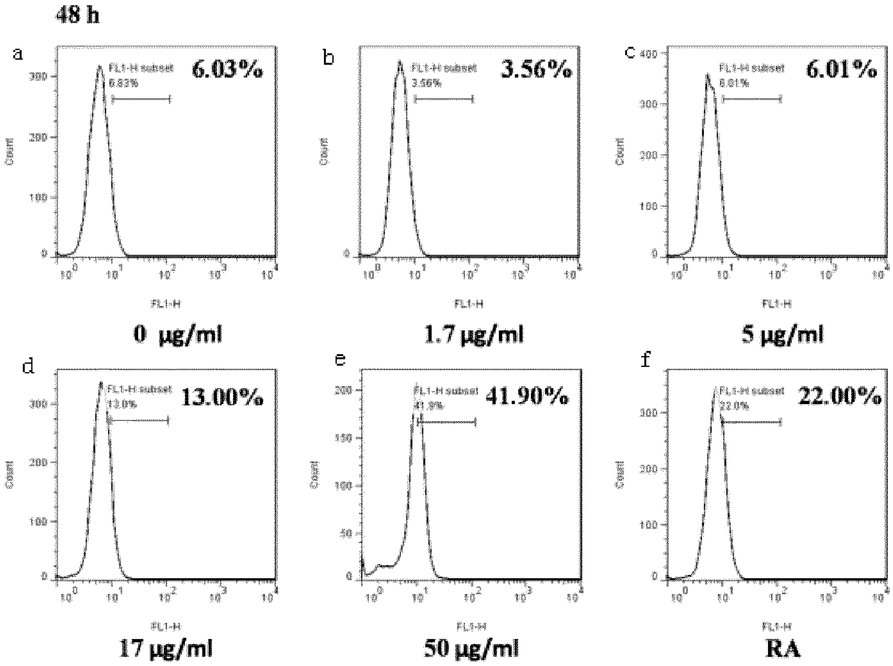
48 h
a
6.03%
0 µg/ml
b
3.56%
1.7 µg/ml
c
6.01%
5 µg/ml
d
13.00%
17 µg/ml
e
41.90%
50 µg/ml
f
22.00%
RA

Fig. 6b

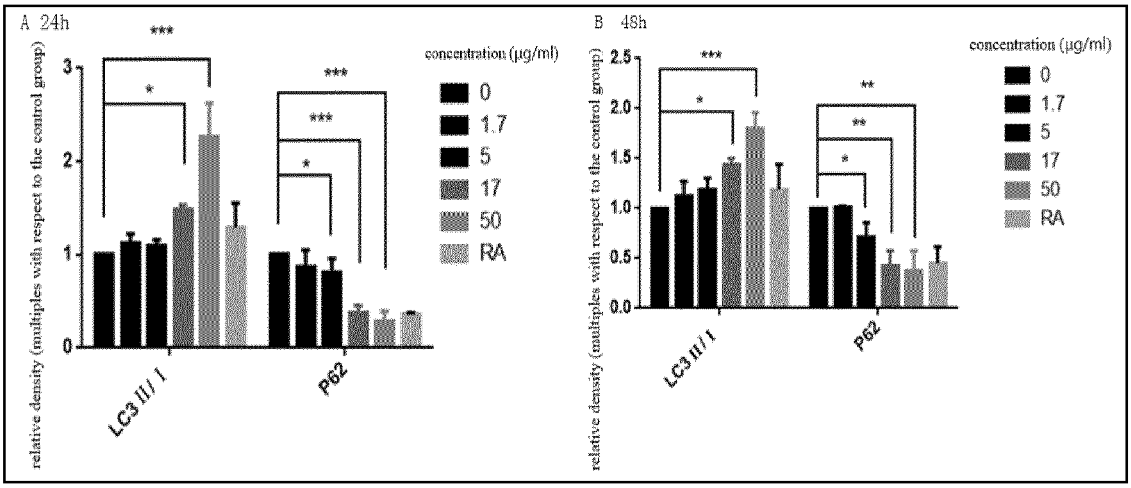
A 24h
B 48h
relative density (multiples with respect to the control group)
concentration (µg/ml)
0
1.7
5
17
50
RA
LC3 II/ I
P62

Fig. 7

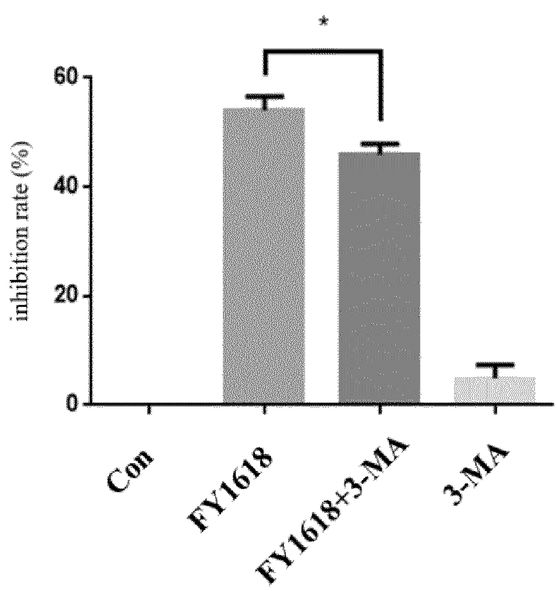
*
60
40
20
0
inhibition rate (%)
Con
FY1618
FY1618+3-MA
3-MA

Fig. 8

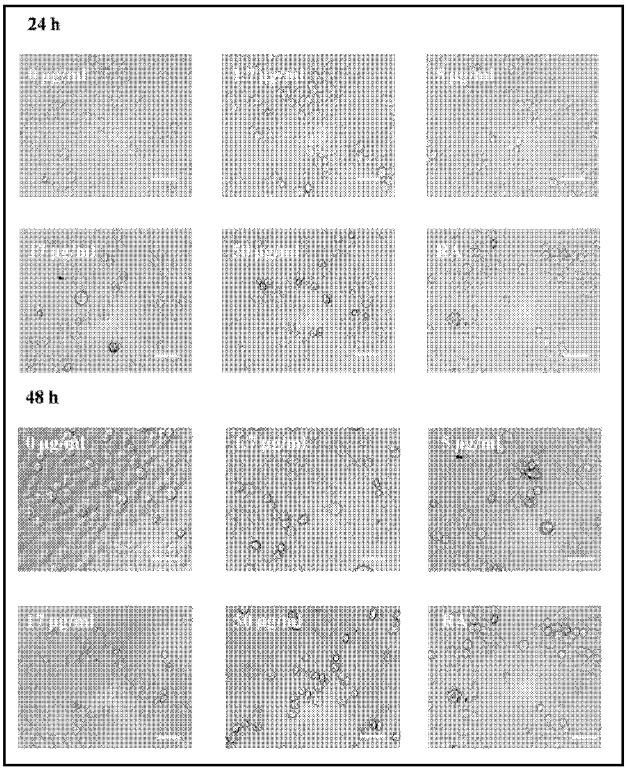
24 h
0 μg/ml
1.7 μg/ml
5 μg/ml
17 μg/ml
50 μg/ml
RA
48 h
0 μg/ml
1.7 μg/ml
5 μg/ml
17 μg/ml
50 μg/ml
RA

Fig. 9

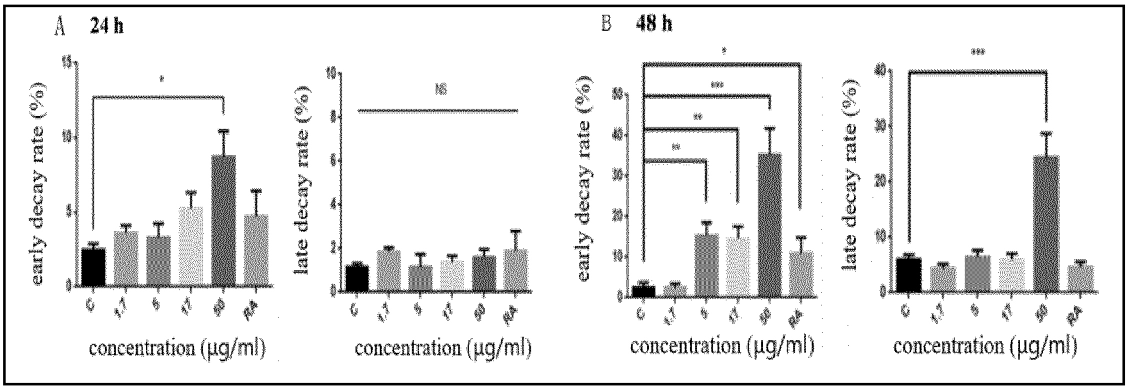


Fig. 10

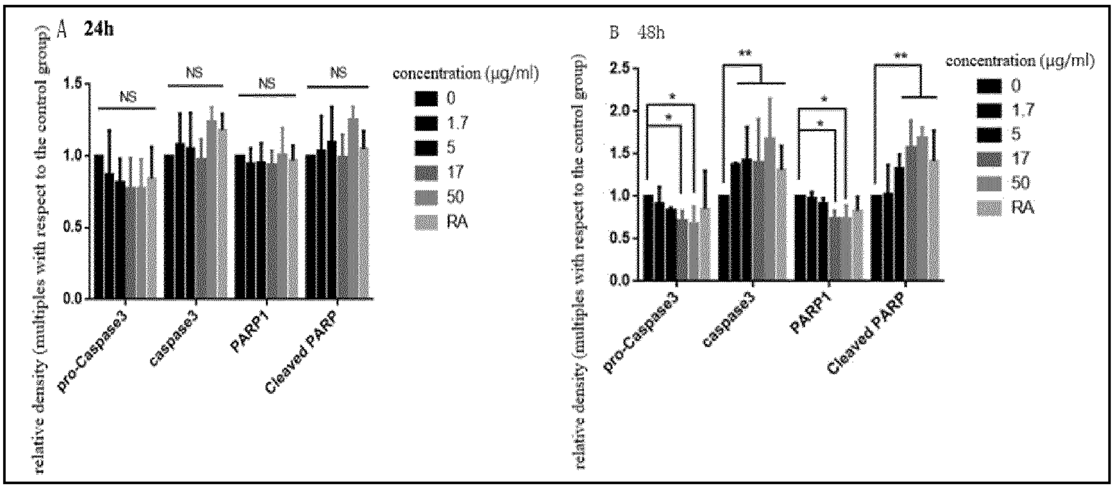


Fig. 11

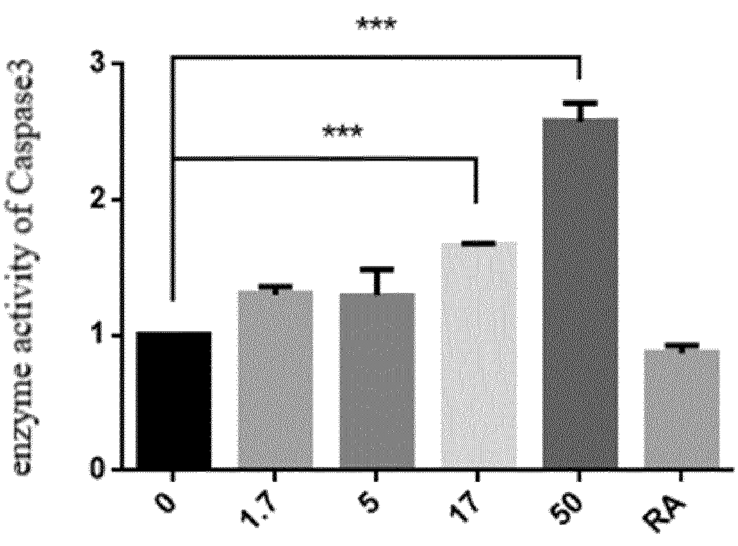


Fig. 12

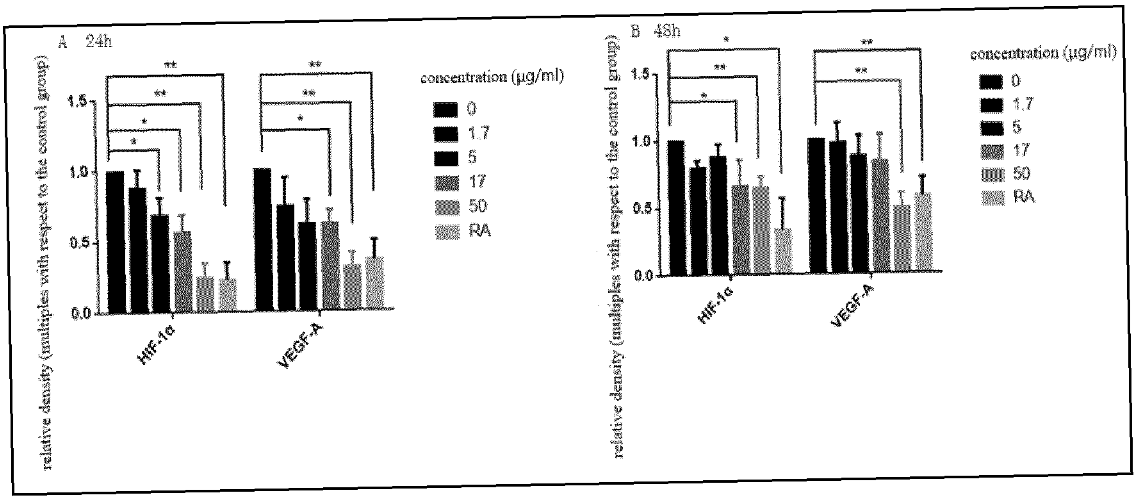
A 24h
relative density (multiples with respect to the control group)
concentration (μg/ml)
0
1.7
5
17
50
RA
HIF-1α
VEGF-A
B 48h
NS

Fig. 13

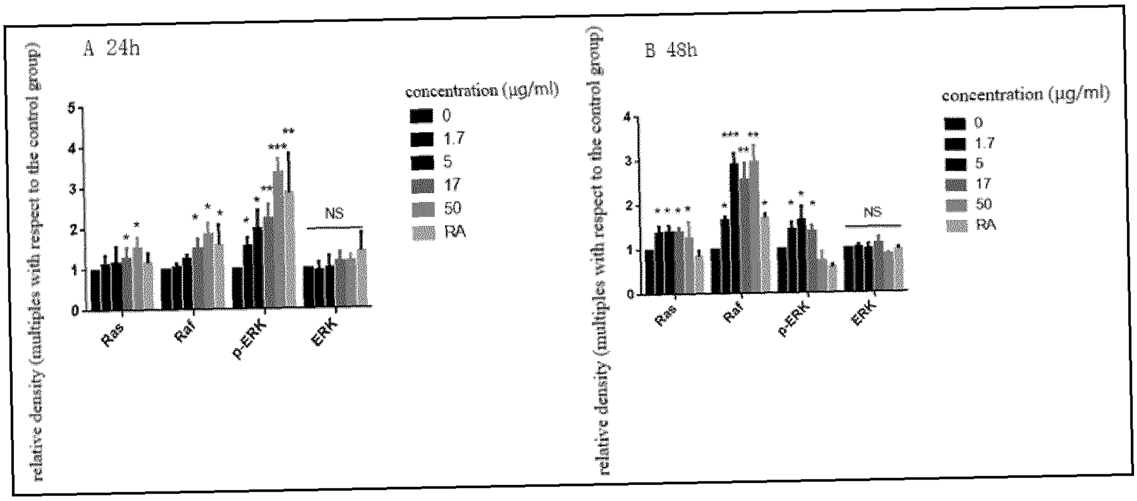
A 24h
relative density (multiples with respect to the control group)
concentration (μg/ml)
0
1.7
5
17
50
RA
Ras
Raf
p-ERK
ERK
NS
B 48h

Fig. 14

## INTERNATIONAL SEARCH REPORT

International application No.
**PCT/CN2019/072411**

**A. CLASSIFICATION OF SUBJECT MATTER**

A61K 31/7048(2006.01)i; A61K 45/06(2006.01)i; A61P 35/00(2006.01)i; A61P 29/00(2006.01)i; A61P 25/28(2006.01)i; A61P 3/10(2006.01)i; A61P 9/00(2006.01)i; A61P 11/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS; DWPI; SIPOABS; VEN; CNTXT; EPTXT; USTXT; WOTXT; CNKI; 中国药物专利数据库, Chinese Pharmaceutical Patent Database; 百度学术搜索, Baidu Scholar Search; ISI-WEB OF SCIENCE; Pubmed: 异戊酰螺旋霉素, 必特螺旋霉素, 螺旋霉素, 可利霉素, 生技霉素, 哺乳动物雷帕霉素靶蛋白, 激酶, 癌, 肿瘤, 白血病, 淋巴瘤, Isovalerylspiramycin, Leucomycin, kelimycin, shengjimycin, Bitespiramycin, ISV, Isovaleryl spiramycin, spiramycin, mTOR, kinase, cancer, tumor, tumour, carcinoma, anticancer, antitumor, antineoplastic, leukemia, lymphadenoma, lymphoid tumor, lymphoma

**C. DOCUMENTS CONSIDERED TO BE RELEVANT**

| Category* | Citation of document, with indication, where appropriate, of the relevant passages | Relevant to claim No. |
|---|---|---|
| PX | WO 2019007368 A1 (SHENYANG FUYANG PHARMACEUTICAL TECH CO., LTD.) 10 January 2019 (2019-01-10)<br>claims 1-14, and description, page 1, paragraph 4 to page 47, paragraph 2 | 1-6, 8-10 |
| X | 杨亚莉等 (YANG, Yali et al.). "HPLC 法分析可利霉素的组分 (Determination of the Components of Bitespiramycin by HPLC)"<br>*药学学报 (Acta Pharmaceutica Sinica),* Vol. 44, No. (10), 31 October 2009 (2009-10-31), ISSN: 0513-4870,<br>page 1184, left column, paragraph 2 to page 1186, left column, paragraph 2 | 1-9 |
| Y | YANG, Yali et al. "HPLC 法分析可利霉素的组分 (Determination of the Components of Bitespiramycin by HPLC)"<br>*药学学报 (Acta Pharmaceutica Sinica),* Vol. 44, No. (10), 31 October 2009 (2009-10-31), ISSN: 0513-4870,<br>page 1184, left column, paragraph 2 to page 1186, left column, paragraph 2 | 8-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 April 2019** | **24 April 2019** |
| Name and mailing address of the ISA/CN<br>**State Intellectual Property Office of the P. R. China**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2019/072411**

**C. DOCUMENTS CONSIDERED TO BE RELEVANT**

| Category* | Citation of document, with indication, where appropriate, of the relevant passages | Relevant to claim No. |
|---|---|---|
| Y | CN 101568343 A (RAMOT AT TEL AVIV UNIVERSITY LTD.) 28 October 2009 (2009-10-28)<br>abstract, claims 1-4 and 7, and description, page 13, paragraph 1 to page 15, paragraph 1 | 8-10 |
| Y | 张涛 (ZHANG, Tao). "二甲双胍对膀胱癌的抑制作用及其机制研究 (Antitumor Effects of Metformin in Bladder Cancer and the Mechanisms)"<br>*中国博士学位论文全文数据库 (电子期刊) 医药卫生科技辑 (Medicine & Public Health, China Doctoral Dissertations Full-Text Database (Electronic Journals)),*<br>No. no. 12, 15 December 2014 (2014-12-15),<br>ISSN: 1674-022X,<br>pages 1-5, and the Chinese abstract | 8-10 |
| X | WO 2019007368 A1 (SHENYANG FUYANG PHARMACEUTICAL TECH CO., LTD.) 10 January 2019 (2019-01-10)<br>claims 1-14, and description, page 1, paragraph 4 to page 47, paragraph 2 | 7 |

**INTERNATIONAL SEARCH REPORT**
**Information on patent family members**

International application No.
**PCT/CN2019/072411**

| Patent document cited in search report | | | Publication date (day/month/year) | Patent family member(s) | | | Publication date (day/month/year) |
|---|---|---|---|---|---|---|---|
| WO | 2019007368 | A1 | 10 January 2019 | None | | | |
| CN | 101568343 | A | 28 October 2009 | EP | 2029147 | A1 | 04 March 2009 |
| | | | | JP | 2009539970 | A | 19 November 2009 |
| | | | | DE | 602007006663 | D1 | 01 July 2010 |
| | | | | EP | 2029147 | B1 | 19 May 2010 |
| | | | | US | 2011306571 | A1 | 15 December 2011 |
| | | | | WO | 2007144876 | A1 | 21 December 2007 |
| | | | | US | 9486467 | B2 | 08 November 2016 |
| | | | | AT | 468119 | T | 15 June 2010 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101785778 A


### Non-patent literature cited in the description


- **FRYDMAN AM et al.** *J Antimicrob Chemother,* 1988, vol. 22 (B), 90-103